# EUROPÄISCHE PATENTSCHRIFT

(11) **EP 2 779 900 B1**
(45) Veröffentlichungstag und Bekanntmachung des Hinweises auf die Patenterteilung: **16.09.2015**
(21) Anmeldenummer: 12784007.2
(22) Anmeldetag: 12.11.2012
(51) Int. Cl.: A61B 5/145, G01N 35/00, G01N 21/64

(54) **ANALAYSEGERÄT ZUM NACHWEIS MINDESTENS EINES ANALYTEN IN EINER PROBE**
ANALYTICAL APPARATUS FOR DETECTING AT LEAST ONE ANALYTE IN A SAMPLE
INSTRUMENT D'ANALYSE POUR LE DÉPISTAGE D'AU MOINS UN ANALYTE DANS UN ÉCHANTILLON

(30) Priorität: 14.11.2011 EP 11189010
(43) Veröffentlichungstag der Anmeldung: 24.09.2014
(73) Patentinhaber: Roche Diagnostics GmbH, 68305 Mannheim (DE); F.Hoffmann-La Roche AG, 4070 Basel (CH)
(72) Erfinder: PETRICH, Wolfgang, 76669 Bad Schönborn (DE); HORN, Carina, 68647 Biblis (DE); STEINKE, Nelli, 68623 Lampertheim (DE); RINGEMANN, Christian, 68165 Mannheim (DE); VON KETTELER, Alexa, 89075 Ulm (DE)
(74) Vertreter: Herzog, Fiesser & Partner Patentanwälte PartG mbB
(86) Internationale Anmeldenummer: PCT/EP2012/072386
(87) Internationale Veröffentlichungsnummer: WO 2013/072275

(56) Entgegenhaltungen:
- EP-A2- 0 355 849
- WO-A1-01/60248
- WO-A1-2005/106435
- WO-A2-03/023356
- DE-A1-102008 056 583

## Beschreibung

### Gebiet der Erfindung

Die Erfindung betrifft ein Analysegerät und ein Verfahren zum Nachweis mindestens eines Analyten in einer Probe. Die Erfindung betrifft weiterhin die Verwendung eines erfindungsgemäßen Analysegeräts zur Vermeidung von Analytmessungen unter Verwendung von Testelementen mit degradierter Testchemie. Derartige Analysegeräte, Verfahren und Verwendungen werden allgemein zum Nachweis eines oder mehrerer Analyten in einer oder mehreren Proben, insbesondere in flüssigen Proben, vorzugsweise in Körperflüssigkeiten, eingesetzt. Ein Einsatzgebiet, auf welches die vorliegende Erfindung jedoch nicht beschränkt ist, ist der Einsatz in der medizinischen Diagnostik, insbesondere der in-vitro Diagnostik. Hierbei können ein oder mehrere Analyte, welche in einem menschlichen oder tierischen Körper vorhanden sein können, in einer Probe einer Körperflüssigkeit nachgewiesen werden, beispielsweise in Blut, interstitielle Flüssigkeit, Speichel, Urin oder in einer Stuhlprobe. Als Analyt kommen insbesondere Stoffe in Betracht, welche direkt oder indirekt am Metabolismus des Körpers beteiligt sein können, insbesondere Glucose, Lactat, Cholesterine oder andere Arten von Analyten oder Analytkombinationen. Die Erfmdung wird, ohne Beschränkung weiterer möglicher Ausgestaltungen, im Folgenden insbesondere bezüglich eines Nachweises von Glucose in einer Körperflüssigkeit beschrieben, insbesondere unter Bezugnahme auf den Nachweis von Blutglucose. Daneben sind auch weitere Einsatzgebiete möglich, wobei auch Einsatzgebiete außerhalb der medizinischen Diagnostik realisierbar sind, beispielsweise in der allgemeinen Analytik oder in der chemischen Verfahrenstechnik.

### Stand der Technik

Aus dem Stand der Technik sind eine Vielzahl von Testelementen und Testverfahren zum Nachweis eines oder mehrerer Analyte in einer Probe bekannt. Hierfür werden in der Regel Testelemente eingesetzt, insbesondere in Zusammenwirkung mit Analysegeräten, welche die Testelemente auswerten. Testelemente weisen in der Regel mindestens eine Testchemie auf, welche mindestens ein Nachweisreagenz zum qualitativen und/oder quantitativen Nachweise des Analyten umfasst. Unter einem Nachweisreagenz ist dabei allgemein, wie nachfolgend noch näher ausgeführt wird, eine chemische Substanz oder ein chemisches Substanzgemisch zu verstehen, welches bei Anwesenheit des mindestens einen Analyten mindestens eine nachweisbare Eigenschaft ändert, insbesondere eine physikalisch und/oder chemisch nachweisbare Eigenschaft. Vorzugsweise findet diese Eigenschaftsänderung spezifisch ausschließlich bei Anwesenheit des mindestens einen nachzuweisenden Analyten statt, nicht hingegen bei Gegenwart anderer Substanzen. Es kann jedoch in der Praxis eine unspezifische Eigenschaftsänderung in einem gewissen Rahmen toleriert werden, bei Anwesenheit anderer chemischer Substanzen, deren Anwesenheit in der Probe der Körperflüssigkeit in der Regel unwahrscheinlich ist und/oder welche lediglich in sehr geringer Konzentration vorhanden sind.

Bei der mindestens einen Eigenschaftsänderung kann es sich beispielsweise um die Änderung einer optisch nachweisbaren Eigenschaft handeln, insbesondere um eine Farbänderung. Beispiele diagnostischer Testelemente mit optischen Nachweisreagenzien sind aus dem Stand der Technik hinlänglich bekannt. Testchemien, welche auch im Rahmen der vorliegenden Erfindung grundsätzlich einsetzbar sind, sind beispielsweise in EP 0 821 234 B1, in WO 2007/012494 A1, in EP 2 093 284 A1 oder in WO 2010/094632 A1 beschrieben. Weiterhin ist in diesen Dokumenten grundsätzlich auch der Aufbau von möglichen Testelementen dargestellt, welche grundsätzlich auch im Rahmen der vorliegenden Erfindung einsetzbar sind. Weitere mögliche Ausgestaltungen einer Testchemie sowie entsprechende Testelemente, welche ebenfalls auch im Rahmen der vorliegenden Erfindung einsetzbar sind, finden sich in WO 2010/052306 A1, in WO 2010/052307 A2 und in WO 2010/094426 A1, EP 1 780 288 A1, WO 2009/103540 A1, WO 2009/015870 A1, US 2007/0026476 A1 oder auf J. Hönes et al, Diabetes Technology and Therapeutics, Vol. 10, Supplement 1, 2008, S-10 bis S-26. Die dort dargestellten Testchemien und Testelemente sind auch im Rahmen der vorliegenden Erfindung grundsätzlich einsetzbar. Auch andere Testchemien und/oder Testelemente sind jedoch alternativ oder zusätzlich verwendbar.

In vielen Fällen umfasst die Testchemie, beispielsweise gemäß den genannten Veröffentlichungen, mindestens ein Enzym und/oder verwendet mindestens einen enzymatischen Nachweis. Beispielsweise können bei einem derartigen enzymatischen Nachweis Ladungsträger erzeugt werden, welche beispielsweise auf einen oder mehrere Indikatorfarbstoffe übertragen werden können oder welche direkt oder indirekt elektrochemisch nachgewiesen werden können. So sind beispielsweise enzymatische Nachweisreaktionen bekannt, bei welchen Ladungsträger auf Reaktionsäquivalente übertragen werden, welche beispielsweise bei der Nachweisreaktion vorübergehend in einer zum Umsetzung des Analyten äquivalenten oder korrespondierenden Menge entstehen können. Diese Reaktionsäquivalente und/oder deren Ladungsträger können nachgewiesen werden, beispielsweise über elektrochemische Nachweisreaktionen, oder es kann wiederum ein Übertrag von Ladungen auf entsprechende Indikatoren erfolgen, beispielsweise Farbstoffe, so dass beispielsweise ein Farbumschlag beobachtet werden kann. Beispiele enzymatischer Nachweisreaktionen, welche auch im Rahmen der vorliegenden Erfindung in der Testchemie eingesetzt werden können, sind in J. Hönes et al, Diabetes Technology and Therapeutics, Vol. 10, Supplement 1, 2008, S-10 bis S-26 beschrieben.

Der Nachweis des Analyten kann bespielsweise elektrochemisch und/oder optisch erfolgen, unter Verwendung der mindestens einen Testchemie. Beispielsweise kann eine Reaktion des nachzuweisenden Analyten mit der Testchemie oder einem Teil derselben zu einer Änderung einer Menge eines nachweisbaren Fluorophors führen, wobei die Menge des Fluorophors mit der Konzentration des Analyten korrelieren kann. Zum Nachweis kann beispielsweise eine für die Menge des Fluorophors charakteristische Messgröße erfasst werden. Derartige Nachweisverfahren sind auch im Rahmen der vorliegenden Erfindung einsetzbar. Insbesondere können dabei Fluoreszenzspektroskopie-Verfahren eingesetzt werden, wie sie beispielsweise in EP 1 780 288 A1 oder in WO 2009/015870 A1 beschrieben werden.

Eine große technische Herausforderung bei bekannten Testelementen stellt deren Stabilität dar. So können beispielsweise Sauerstoff und Feuchtigkeit die Qualität der Testchemie oder von Teilen derselben beeinträchtigen. Aus dem Stand der Technik sind erhebliche Anstrengungen bekannt, um die Testchemie gegenüber derartigen Einflüssen zu stabilisieren und auf diese Weise beispielsweise Anforderungen an eine Lagerung der Testelemente zu verringern und eine Langzeitstabilität zu erhöhen. Beispielsweise wird in der bereits genannten WO 2007/012494 A1 eine Testchemie mit stabilen NAD/NADH-Derivaten beschrieben. In EP 2 093 284 A1 wird eine Stabilisierung von Dehydrogenasen mit stabilen Coenzymen beschrieben.

Aus EP 1 189 064 A1 ist ein Verfahren zum Kontrollieren einer Gebrauchstauglichkeit von Analyseelementen bekannt. Dabei wird die Abweichung eines Quotienten aus einem Kontrollwert und einem ersten Standardreferenzwert gegenüber einem ersten Bezugsquotienten, der aus einem Kontrollbezugswert und einem ersten Standardreferenzwert gebildet wird, überprüft. Ein kontrolliertes Analyseelement wird zurückgewiesen, wenn die Abweichung außerhalb eines vorgegebenen Toleranzbereichs liegt. Unter anderem wird dabei vorgeschlagen, die Gebrauchsfähigkeit eines Analyseelements anhand einer so genannten Trockenleerwertmessung eines Testfelds zu überprüfen, d.h. einer optischen Messung eines noch nicht mit Probenflüssigkeit benetzten Testfelds. Zur Durchführung des Kontrollverfahrens wird vorgeschlagen, dass das Analyseelement neben einem zur Kontrolle seiner Gebrauchstauglichkeit und zur Durchführung der Analyse dienenden Testfeld vorteilhafter Weise ein integriertes Referenzkontrollmittel aufweist. Das in EP 1 189 064 A1 vorgeschlagene Verfahren weist jedoch in der Praxis zahlreiche Herausforderungen auf. So ist mittels der Trockenleerwertmessung, bei welcher eine Remission des Testfelds vor einer Benetzung mit der Probe gemessen wird, lediglich eine Grobkontrolle einer Alterung des Analyseelements erkennbar. Auf diese Weise lässt sich beispielsweise anhand einer Verfärbung des Testfelds, welche beispielsweise auf eine Verfärbung eines in dem Testfeld enthaltenen Farbstoff zurückzuführen ist, innerhalb sehr grober Grenzen ein Ausschluss von erheblich degradierten Testelementen vornehmen. Weiterhin stellt die Bereitstellung des integrierten Referenzkontrollmittels technische Anforderungen an die Gestaltung der Testelemente, welche nicht in allen Fällen einfach und kostengünstig realisierbar sind.

Aus EP 2 221 608 A1 ist weiterhin ein Testverfahren zur Untersuchung einer Körperflüssigkeit mittels eines Testbandes bekannt. Um eine Messsicherheit zu erhöhen, wird vorgeschlagen, dass ein Kontrollwert aus einer zeitlichen und/oder wellenlängenabhängigen Änderung von Messsignalen über die Dauer eines Messzeitintervalls ermittelt wird. Anhand dieses Kontrollwerts werden Messsignale nach der Messung als gültig verarbeitet oder als fehlerhaft verworfen. Unter anderem wird dabei auch vorgeschlagen, aus einer Leerwertmessung noch unbenutzter Testfelder einen Testfeldkontrollwert zu ermitteln und die Verwertbarkeit des Testfelds durch Vergleich mit einem Chargenkontrollwert oder einen Einfluss der Lagerzeit auf das Testmaterial zu erkennen. Dabei wird beispielsweise vorgeschlagen, den Chargenkontrollwert auf einem dem Testband zugeordneten Speichermittel zu speichern. Auch dieses in EP 2 221 608 A1 vorgeschlagene Verfahren weist in der Praxis jedoch einige technische Herausforderungen auf. So ist dieses Verfahren beispielsweise daran gebunden, dass ein Chargenkontrollwert ermittelt und den Testelementen beigefügt wird. Weiterhin sind auch in diesem Fall aufgrund der Remissionsmessung eines Trockenleerwerts in der Regel lediglich grobe Degradationen einer Testchemie erkennbar, welche insbesondere wiederum auf eine Degradation eines Farbstoffs und eine damit verbundene Farbänderung der Testfelder zurückzuführen sind. Degradationen, welche auf diese Weise nicht erkennbar sind und welche beispielsweise nicht zu einer Farbveränderung der Testfelder führen, lassen sich auf diese Weise nur vergleichsweise schwer erkennen und ausschließen.

In WO 01/60248 A1 werden Verfahren und Vorrichtungen zur nicht-invasiven Messung von Analytkonzentrationen, insbesondere Glucosekonzentrationen, in Gewebe beschrieben. Dabei wird ein Target innerhalb des Gewebes des Patienten optisch angeregt, welches nicht die Glucose selbst ist, dessen Fluoreszenz jedoch mit der Glucosekonzentration korreliert. Unter anderem wird dabei vorgeschlagen, als Target PDCCL (pepsin digestible collagen cross links) zu verwenden. Die Glucose selbst weist eine geringe Eigenfluoreszenz auf, wohingegen sich die Fluoreszenz von PDCCL in Abhängigkeit vom Glucoselevel des Patienten ändert. Weiterhin wird offenbart, dass die Fluoreszenz des Targets abhängig sein kann von bestimmten Effekten, wie beispielsweise Alter, UV-Belastung, Hautfarbe oder anderen Effekten. Dementsprechend wird vorgeschlagen, Fluoreszenzsignale der Haut bei Bestrahlung im ultravioletten Spektralbereich (UVA) zu verwenden, um den Zustand der Kollagenmatrix zu bewerten und zu berücksichtigen.

In DE 10 2008 056583 A1 werden ein Verfahren und eine Vorrichtung zur Feststellung einer Reagenzienqualität beschrieben. Dabei wird ein Trägerelement mit einem Testmaterial zeitgleich mit bestimmten Objekten durch Behandlungsstationen hindurchgeführt. Die dabei verursachten Veränderungen des Testmaterials werden erfasst und mit Referenzdaten verglichen. Unter anderem wird vorgeschlagen, durch die Behandlung verursachte charakteristische Eigenschaften des Testmaterials mittels Fluoreszenz zu erfassen.

In WO 03/023356 A2 werden Vorrichtungen und Verfahren zur nicht-invasiven Messung von Analyt-Konzentrationen in vivo beschrieben. Dabei wird ein optischer Koppler verwendet, um eine Hautoberfläche mit einer Vorrichtung zu verbinden, welche eine Mehrzahl von Zonen umfasst. Diese Zonen umfassen Flächen für eine Mehrzahl von Zwecken, einschließlich einer Kalibration der Vorrichtung, eines Ablesens der Hautoberfläche und einer Schutzfunktion für die Vorrichtung.

Aus dem Stand der Technik ist weiterhin allgemein die Verwendung von Fluorophoren zür automatischen Messung von Fluorezenz, beispielsweise in EP 0 355 849 A2, und zur Erkennung von Glucosekonzentrationen in Teststreifen bekannt. Diesbezüglich kann beispielsweise auf EP 1 780 288 A1 oder auf WO 2009/015870 A1 verwiesen werden. Glucose-induzierte Veränderungen in der Fluoreszenz von Proteinen und anderen Fluorophoren werden beispielsweise in J. C. Pickup et al., Biosensors and Bioelectronics 20 (2005) 2555, zur Glucosedetektion herangezogen. In C. M. Moore, Biomacromolecules 5 (2004) 1241, Seite 1243, wird weiterhin beschrieben, dass die Lebensdauer von Alkohol-Dehydrogenase durch Messung des bei einer Nachweisreaktion entstehenden Coenzyms NADH gemessen werden kann. In V. Scognamiglio, Journal of Fluorescence, 14 (3) (2004) 491 und in G. Mendoza-Hernandez, Biochimica et Biophysica Acta 1478 (2000) 221, finden sich allgemein Hinweise auf die Änderung intrinsischer Fluoreszenzeigenschaften von Proteinen mit deren Konformation, beispielsweise induziert durch Harnstoff. In M. V. Duffelen Biophysical Journal 87 (2004) 1767, wird eine Autofluoreszenz von Proteinen mit Tryptophan verbunden, dessen Anregungswellenlänge bei 295 nm liegt und dessen Emissionsspektrum im Intervall von 305 bis 400 nm detektiert wird. In S. R. Bhaumik, Physiological Chemistry in Physics and Medicine NMR 31 (1999) 85, wird über die Verschiebung einer Fluoreszenzemission bei 340, 347 und 354 nm unter einer Denaturierung eines Proteins berichtet. Auch in dem erwähnten G. Mendoza-Hernandez, Biochim. Biophys. Acta 1478 (2000) 221, wird auf die Änderung von Emissionseigenschaften des in dem Protein befindlichen Tryptophans hingewiesen. Ähnlich beschreibt W. Hilt, Biochim. Biophys, Acta 1076 (1991) 298 eine Änderung einer Fluoreszenzemission von GlucDH-S, wobei auch Absorptionsspektren beschreiben werden. In der genannten Veröffentlichung von G. Mendoza-Hernandez, Biochim. Biophys. Acta 1478 (2000) 221, wird weiterhin darauf hingewiesen, dass eine Degeneration von GlucDH mit Urea reversibel ist. Eine Reversibilität einer Dissoziation und Denaturierung von GlucDH wird auch in H. E. Pauly, Biochemistry 16 (21) 1977, 4599, beschrieben. Dort wird weiterhin ausgeführt, dass eine Blauverschiebung einer Absorption bei Dissoziation beobachtbar ist. Auch in T. Yamazaki et al., Applied Biochemistry and Biotechnology 77 - 79 (1999) 325, werden Absorptionsmessungen an GlucDH beschrieben. Dabei wird bei 409 nm ein Absorptionspeak beobachtet. Eine Temperaturbelastung des Enzyms führt zum Verschwinden dieses Absorptionspeaks. Weiterhin wird eine Fluoreszenz beobachtet, welche sich bei einer Temperaturbehandlung erniedrigt und welche auf einen unbekannten Co-Faktor zurückgeführt wird. In K. Inose, Biochim. Biophys. Acta 1645 (2003) 133-138, zeigt sich, dass die Autofluoreszenz durch den Co-Faktor FAD verursacht wird.

Trotz der mit den bekannten Verfahren, Vorrichtungen und Testchemien erzielten Fortschritte besteht nach wie vor bei den bekannten Nachweisverfahren eine Restunsicherheit hinsichtlich möglicher Alterungserscheinungen der Testelemente. Gelöst wird diese Problematik in der Praxis dadurch, dass Testelemente, welche beispielsweise als Einzelteststreifen oder als Testelemente mit mehreren Testchemiefeldern vertrieben werden, ein Haltbarkeitsdatum aufweisen. Über eine entsprechende Kodierung können Analysegeräte auch beispielsweise erkennen, ob dieses Haltbarkeitsdatum überschritten wurde und entsprechend eine Verwendung derartig gealterter Testelemente verhindern. Dennoch besteht, auch vor Ablauf der nominellen Lebensdauer, das Risiko, dass defekte oder gealterte Testelemente für eine Messung herangezogen werden können. So werden beispielsweise Testelemente in Behältnissen geliefert, in welchen ein Trockenmittel enthalten ist, um eine feuchtigkeitsarme Atmosphäre für die Lagerung zu gewährleisten. Ein Benutzer ist beispielsweise aufgefordert, dieses Behältnis unmittelbar nach Entnahme eines Teststreifens wieder zu verschließen. Insbesondere bei Benutzern mit Demenzerscheinungen oder auch bei Kindern kann jedoch in der Praxis nicht immer gewährleistet werden, dass eine derartig korrekte Behandlung der Testelemente auch tatsächlich erfolgt, so dass eine Messung unter Verwendung degradierter Testelemente nicht in allen Einzelheiten ausgeschlossen werden kann.

### Aufgabe der Erfindung

Es ist daher eine Aufgabe der vorliegenden Erfindung, ein Analysegerät und ein Verfahren zum Nachweis mindestens eines Analyten in einer Probe bereit zu stellen, welche die Nachteile bekannter Analysegeräte und Verfahren zumindest weitgehend vermeiden. Insbesondere soll eine Verwendung degradierter Testelemente möglichst vermieden werden, sei es nun eine Degradation aufgrund einer Überschreitung einer Lagerlebensdauer oder eine Degradation aufgrund einer fehlerhaften Behandlung oder Lagerung der Testelemente.

### Offenbarung der Erfindung

Diese Aufgabe wird durch ein Analysegerät und ein Verfahren mit den Merkmalen der unabhängigen Patentansprüche gelöst. Vorteilhafte Weiterbildungen der Erfindung, welche einzeln oder in beliebiger Kombination realisierbar sind, sind in den abhängigen Patentansprüchen dargestellt.

In einem ersten Aspekt der vorliegenden Erfindung wird ein Analysegerät zum Nachweis mindestens eines Analyten in einer Probe vorgeschlagen. Das Analysegerät ist eingerichtet, um mindestens eine Analytmessung durchzuführen, wobei bei der Analytmessung mindestens eine durch Anwesenheit des Analyten veränderliche Eigenschaft mindestens einer Testchemie eines Testelements erfasst wird, insbesondere eine elektrische und/oder optische Eigenschaft.

Unter einem Analyten wird allgemein im Rahmen der vorliegenden Erfindung eine beliebige Substanz oder Substanzkombination verstanden, welche qualitativ oder quantitativ nachgewiesen werden soll. Wie oben ausgeführt, kann es sich bei diesem mindestens einen Analyten, wobei vorzugweise genau ein Analyt oder eine Gruppe bestimmter Analyte spezifisch nachgewiesen werden, insbesondere um mindestens eine Substanz handeln, welche direkt oder indirekt an einem Stoffwechsel eines menschlichen oder tierischen Körpers beteiligt ist. Insbesondere kann es sich um mindestens einen Metaboliten handeln. Beispiele von Analyten, welche einzeln oder in beliebiger Kombination nachgewiesen werden können, sind Glucose, insbesondere Blutglucose, Harnsäure, Ethanol, Lactat, Cholesterin. Auch andere Analyte sind jedoch grundsätzlich nachweisbar.

Bei der mindestens einen Probe kann es sich insbesondere um eine flüssige Probe handeln. Insbesondere kann es sich um eine Körperflüssigkeit handeln. Beispielsweise kann die flüssige Probe ausgewählt sein aus Vollblut, Blutplasma, interstitieller Flüssigkeit, Speichel, Urin oder andern Arten von Körperflüssigkeiten. Alternativ oder zusätzlich zu einer Körperflüssigkeit kann die flüssige Probe jedoch auch mindestens eine weitere Flüssigkeit umfassen, beispielsweise mindestens eine Kontrolllösung. Derartige Kontrolllösungen können beispielsweise mindestens einen nachzuweisenden Analyten in einer vorgegebenen Konzentration in mindestens einem Lösungsmittel oder Lösungsmittelgemisch aufweisen, beispielsweise Glucose in einer vorgegebenen Konzentration in einem Lösungsmittel wie beispielsweise Wasser.

Unter einem Nachweis ist allgemein im Rahmen der vorliegenden Erfindung ein Vorgang zu verstehen, bei welchem mindestens eine Information generiert wird, die qualitativ oder quantitativ Rückschluss auf das Vorhandensein oder Nicht-Vorhandensein des Analyten in der Probe und/oder eine Menge oder Konzentration der Analyten in der Probe schließen lässt. Diese Information kann beispielsweise direkt an einen Benutzer vermittelt werden und/oder kann in elektronischer Form vorliegen, beispielsweise in einem Datenspeicher und/oder durch Weitergabe an ein von dem Analysegerät getrenntes Gerät.

Unter einer Analytmessung ist im Rahmen der vorliegenden Erfindung ein Messvorgang zu verstehen, bei welchem mindestens eine erfassbare Größe erfasst wird, beispielsweise mindestens eine Messgröße, welche dem Nachweis des Analyten dient. Beispielsweise kann die erfasste Größe eine physikalische Messgröße umfassen, wie beispielweise eine optische Messgröße, insbesondere ausgewählt aus einem Farbeindruck, einer Lumineszenz und einer Lumineszenzlebensdauer, und/oder eine elektrochemische Größe, beispielsweise eine Spannung und/oder einen Strom. Bezüglich möglicher Ausgestaltungen der Analytmessungen kann beispielsweise auf den oben genannten Stand der Technik sowie beispielsweise allgemein auf bekannte optische und/oder elektrochemische Nachweisverfahren zur Analytmessung verwiesen werden.

Das Analysegerät kann mindestens eine Analytmessvorrichtung aufweisen, um die Analytmessung durchzuführen. Diese Analytmessvorrichtung kann beispielsweise mindestens ein optisches und/oder mindestens ein elektrisches Messgerät aufweisen, beispielsweise um eine optische Messung und/oder eine elektrochemische Messung durchzuführen. Beispiele derartiger Analytmessvorrichtungen werden unten noch näher erläutert.

Bei der Analytmessung wird mindestens eine durch Anwesenheit des Analyten veränderliche Eigenschaft mindestens einer Testchemie eines Testelements erfasst, insbesondere eine elektrische und oder optische Eigenschaft.

Unter einer Testchemie ist dabei allgemein im Rahmen der vorliegenden Erfindung eine Substanz oder ein Substanzgemisch zu verstehen, welches eingerichtet ist, um bei Anwesenheit des Analyten mindestens eine nachweisbare veränderliche Eigenschaft zu verändern, beispielsweise eine physikalisch nachweisbare Eigenschaft. Insbesondere kann die Testchemie eingerichtet sein, um bei Anwesenheit des Analyten mindestens eine von der Anwesenheit des Analyten abhängige Eigenschaft zu ändern. Dabei bestehen mehrere Möglichkeiten. So kann sich beispielsweise die mindestens eine Eigenschaft zwischen zwei Zuständen ändern, wobei ein Zustand eintritt, wenn der Analyt anwesend ist, und ein anderer Zustand, wenn der Analyt abwesend ist. Alternativ oder zusätzlich kann sich die mindestens eine Eigenschaft auch stufenweise oder kontinuierlich ändern, wobei die Eigenschaft stufenweise oder kontinuierlich in Abhängigkeit von einer Konzentration des Analyten mehrere Zustände einnehmen kann, beispielsweise indem die Eigenschaft eine Funktion der Konzentration des Analyten ist. Verschiedene Ausgestaltungen sind möglich.

Bezüglich möglicher Testchemien kann insbesondere auf die obige Beschreibung des Standes der Technik verwiesen werden. Zudem werden nachfolgend bevorzugte Ausgestaltungen der Testchemie weiter beschrieben. Insbesondere kann es sich bei dieser mindestens einen Eigenschaft um eine elektrische und/oder optische Eigenschaft handeln, welche mit einer entsprechenden Analytmessvorrichtung erfasst werden kann. Für den Nachweis elektrischer Eigenschaften kann beispielsweise eine elektrische Messvorrichtung vorgesehen sein, beispielsweise eine Spannungsmessvorrichtung und/oder eine Strommessvorrichtung. Für den Nachweis optischer Eigenschaften kann beispielsweise mindestens ein optischer Analytdetektor vorhanden sein. Ausführungsbeispiele werden unten noch näher beschrieben.

Insofern kann das Analysegerät beispielsweise bekannten Analysegeräten entsprechen. Zur Lösung der oben beschriebenen Problematik wird weiterhin vorgeschlagen, dass das Analysegerät weiterhin eingerichtet ist, um mindestens eine Qualitätsmessung an der Testchemie durchzuführen, wobei bei der Qualitätsmessung mindestens eine Eigenlumineszenz der Testchemie erfasst wird und aus der Eigenlumineszenz auf einen Qualität der Testchemie geschlossen wird, insbesondere auf eine Degradation.

Im Unterschied zu bekannten Analysegeräten, welche ausschließlich der Analytmessung dienen, ist das vorgeschlagene Analysegerät also weiterhin eingerichtet, um die Qualitätsmessung durchzuführen. Unter einer Qualitätsmessung wird dabei allgemein im Rahmen der vorliegenden Erfindung ein Vorgang verstanden, bei welcher qualitativ oder quantitativ die Qualität der Testchemie erfasst wird.

Unter einer Qualität der Testchemie ist allgemein im Rahmen der vorliegenden Erfindung eine Information zu verstehen, welche Aufschluss über einen Zustand der Testchemie gibt. Diese mindestens eine Information umfasst eine Information über einen Alterungszustand der Testchemie, insbesondere eine Information über eine Degradation oder einen Degradationszustand der Testchemie. So kann diese mindestens eine Information beispielsweise digitaler Natur sein und kann beispielsweise die Information "Qualität in Ordnung" oder "Qualität nicht in Ordnung" umfassen. Eine derartige Information kann beispielsweise anhand eines oder mehrerer Schwellwerte ermittelt werden. So kann beispielsweise bei der Qualitätsmessung mindestens ein Qualitätsmesswert generiert werden, beispielsweise in Form eines entsprechenden Signals und/oder in Form einer entsprechenden elektronischen Information, wobei dieser mindestens eine Qualitätsmesswert beispielsweise mit einem oder mehreren Schwellwerten verglichen wird, um die Information der Qualität zu generieren. Alternativ oder zusätzlich zu einer rein digitalen Qualität kann die Qualität auch eine Mehrzahl von Informationen enthalten, so dass beispielsweise die Qualität auch quantifiziert werden kann. So umfasst die Qualität mindestens eine Qualitätsinformation, welche, beispielsweise auf einer vorgegebenen Skala, die Degradation oder einen Alterungszustand der Testchemie quantifiziert.

So kann beispielsweise das Analysegerät derart eingerichtet sein, dass zuvor, vor Durchführung der Qualitätsmessung, eine oder mehrere Vergleichsinformationen generiert werden, was innerhalb des Analysegeräts oder auch extern erfolgen kann. Diese Vergleichsinformationen können beispielsweise in dem Analysegerät abgespeichert sein. Allgemein kann bei der Qualitätsmessung mindestens ein Qualitätsmesswert generiert werden, welcher beispielsweise mit der einen oder den mehreren Vergleichsinformationen verglichen werden kann, beispielsweise mit einer oder mehreren Schwellwerten. Auf diese Weise kann die mindestens eine Information der Qualität erzeugt werden, beispielsweise als Ergebnis dieses mindestens einen Vergleichs. Wie oben ausgeführt, kann diese Information digitaler Natur sein oder kann auch beispielsweise mehrere Abstufungen umfassen, beispielsweise indem, entsprechend der Ergebnisse der Qualitätsmessung, die Qualität der Testchemie in eine oder mehrere kontinuierliche oder diskontinuierliche Kategorien eingestuft wird. Die Information über diese Einstufung kann das Ergebnis der Qualitätsmessung sein.

Unter einer Alterung und insbesondere einer Degradation der Testchemie kann allgemein im Rahmen der vorliegenden Erfindung eine beliebige Änderung der Testchemie oder eines Teils der Testchemie verstanden werden, welche einen Einfluss auf die Analytmessung haben kann. Hierbei kann es sich um chemische Änderungen, wie beispielsweise eine unerwünschte Oxidation und/oder Einlagerung von Wasser, oder auch um physikalische Änderungen handeln, wie beispielsweise um so genannte Konformationsänderungen, Kristallisationen oder ähnliche Effekte.

Unter einer Eigenlumineszenz der Testchemie ist dabei allgemein eine Lumineszenz der Testchemie, also eine Phosphoreszenz und/oder eine Fluoreszenz, zu verstehen, welche von der Testchemie, ggf. unter Zusammenwirkung mit weiteren Elementen des Testelements wie beispielsweise dem Trägerelement, emittiert werden kann, wenn keine Probe auf die Testchemie aufgebracht ist. Eine derartige Eigenlumineszenz kann also beispielsweise vor einem Aufbringen der Probe auf die Testchemie erfasst werden. Zur Erfassung einer Eigenlumineszenz kann die Testchemie beispielsweise mit Anregungslicht mit einer oder mehreren Wellenlängen bestrahlt werden und es kann eine dabei auftretende Lumineszenz, zeitgleich oder zeitversetzt zu der Bestrahlung, mittels eines geeigneten Detektors erfasst werden. Insbesondere kann die Eigenlumineszenz eine Eigenfluoreszenz der Testchemie umfassen.

Zur Durchführung der Qualitätsmessung kann das Analysegerät beispielsweise eine Qualitätsmessvorrichtung umfassen. Diese Qualitätsmessvorrichtung kann beispielsweise mindestens einen Qualitätsdetektor umfassen, welcher mindestens eine aktuelle Eigenschaft der Testchemie erfasst, aus welcher auf die Qualität der Testchemie geschlossen werden kann. Beispielsweise kann dabei mindestens ein Qualitätsmesswert generiert werden. Diese mindestens eine erfassbare Eigenschaft, welche von der Qualitätsmessvorrichtung erfasst wird, kann insbesondere verschieden sein von der veränderlichen Eigenschaft, welche bei der Analytmessung erfasst wird. So können beispielsweise bei der Analytmessung und bei der Qualitätsmessung jeweils elektrische und/oder optische Eigenschaften der Testchemie erfasst werden, welche sich jedoch vorzugsweise unterscheiden. Auch wenn beispielsweise in beiden Fällen optische Eigenschaften erfasst werden, so kann es sich doch insbesondere um unterschiedliche optische Eigenschaften handeln, beispielsweise, wie unten noch näher ausgeführt wird, bei der Analytmessung um eine Remissionsmessung und/oder eine Messung eines Farbumschlags und bei der Qualitätsmessung um eine Messung einer Fluoreszenz. Alternativ oder zusätzlich können auch beispielsweise Fluoreszenzmessungen in unterschiedlichen Spektralbereichen durchgeführt werden. Beispiele werden unten noch näher erläutert.

Mittels der Qualitätsmessung kann die oben beschriebene Problematik bekannter Analysegeräte auf verschiedenen Weisen vermieden werden. So kann die Qualitätsmessung beispielsweise herangezogen werden, um mindestens ein Ergebnis der Analytmessung zu bewerten und/oder zu überarbeiten, beispielsweise zu modifizieren. Beispielsweise kann mittels der Qualitätsmessung das Ergebnis der Analytmessung kalibriert werden. Beispielsweise kann mindestens eine Kalibrationsinformation in dem Analysegerät hinterlegt sein, welche, unter Berücksichtigung des Ergebnisses der Analytmessung und unter Berücksichtigung des Ergebnisses der Qualitätsmessung ein Messergebnis generiert, welches den Analyten in der Probe qualifiziert oder quantifiziert. Alternativ oder zusätzlich kann das Analysegerät jedoch auch eingerichtet sein, um, entsprechend dem Ergebnis der Qualitätsmessung, die Analytmessung zu ermöglichen oder zu verhindern, oder um eine Ausgabe des Ergebnisses der Analytmessung an einen Benutzer oder ein anderes Gerät entsprechend dem Ergebnis der Qualitätsmessung zu ermöglichen oder zu verhindern. So kann beispielsweise in dem Analysegerät mindestens eine Qualitätsschwelle vorgegeben sein, mit welcher die bei der Qualitätsmessung ermittelte Qualität verglichen wird. Auf diese Weise kann beispielsweise ein Ergebnis "Qualität in Ordnung" oder "Qualität nicht in Ordnung" generiert werden. Entsprechend dieses Ergebnisses kann beispielsweise eine nachfolgende Analytmessung unter Verwendung der Testchemie freigegeben oder verhindert werden, oder es kann, wenn die Analytmessung bereits durchgeführt wurde, eine Bewertung des Ergebnisses erfolgen, beispielsweise indem dieses Ergebnis erst gar nicht an einen Benutzer oder ein anderes Gerät übermittelt wird oder indem das Ergebnis beispielsweise mit einem entsprechenden Hinweis, beispielsweise einem Warnhinweis, übermittelt wird. Verschiedene Ausgestaltungen sind möglich.

Unter einem Testelement ist im Rahmen der vorliegenden Erfindung allgemein ein Element zu verstehen, welches mindestens eine Testchemie gemäß der obigen Definition umfasst. Das Testelement umfasst mindestens ein Trägerelement, auf welches die mindestens eine Testchemie aufgebracht ist und/oder in welches die mindestens eine Testchemie eingebracht ist. Dieses mindestens eine Trägerelement kann beispielsweise ganz oder teilweise hergestellt sein aus einem Material ausgewählt aus der Gruppe bestehend aus einem Kunststoffmaterial, einem Papiermaterial, einem Keramikmaterial und einem Laminatmaterial. Auch andere Ausgestaltungen sind möglich. Das Testelement kann ein oder mehrere Felder umfassen, in denen die Testchemie auf das Trägerelement aufgebracht und/oder in das Trägerelement eingebracht ist. Das Testfeld ist ein Bereich, in welchem mindestens eine zusammenhängende Schicht der Testchemie auf das Trägerelement aufgebracht oder in das Trägerelement eingebracht ist.

Das Testelement kann ein oder mehrere derartiger Testfelder umfassen. Diese Testfelder können beispielsweise nebeneinander auf dem Trägerelement oder in dem Trägerelement angeordnet sein. Das Trägerelement kann beispielsweise streifenförmig, scheibenförmig oder bandförmig ausgestaltet sein. So können beispielsweise Teststreifen oder Testbänder verwendet werden. Beispiele werden unten noch näher erläutert.

Das Testelement kann neben der mindestens einen Testchemie und dem mindestens einen Trägerelement weitere Elemente umfassen. So kann das Testelement beispielsweise ein Schichtaufbau umfassen, wobei beispielsweise die mindestens eine Testchemie in Form einer oder mehrerer Testchemieschichten auf das Trägerelement aufgebracht ist. Zusätzlich kann mindestens eine weitere Schicht enthalten sein, beispielsweise eine Abtrennschicht und/oder eine Reflektionsschicht. So kann beispielsweise ein Testaufbau generiert werden, bei welchem, auf das Trägerelement, zunächst mindestens eine Schicht der Testchemie aufgebracht ist und, darauf folgend, mindestens eine Abtrennschicht und/oder Reflektionsschicht, wobei die Abtrennschicht und/oder Reflektionsschicht beispielsweise zum Abtrennen von unerwünschten Bestandteilen der Probe dient, wie beispielsweise zum Abtrennen von roten Blutkörperchen, bevor diese in die Testchemie gelangen und dort beispielsweise einen optischen Nachweis des Analyten stören können. Weiterhin kann die Abtrennschicht und/oder Reflektionsschicht beispielsweise ein oder mehrere Pigmente umfassen, welche beispielsweise reflektive Eigenschaften aufweisen, beispielsweise weiße Pigmente wie Titandioxidpartikel. So kann beispielsweise ein Schichtaufbau entstehen, bei welchem die Oberfläche der mindestens einen Abtrennschicht und/oder Reflektionsschicht, welche der Testchemieschicht abgewandt ist, als Probenaufgabeseite dient. Der Nachweis des mindestens einen Analyten kann beispielsweise durch das Trägerelement hindurch erfolgen, also beispielsweise von einer der Probenaufgabeseite gegenüberliegenden Seite. So kann das Trägerelement beispielsweise optional ganz oder teilweise optisch transparent ausgestaltet sein, beispielsweise optisch transparent für mindestens einen in die Testchemie eingestrahltes Anregungslicht und/oder Transparent für mindestens ein von der Testchemie reflektiertes und/oder emittiertes Nachweislicht, wobei unter einer Transparenz beispielsweise eine Transparenz von mindestens 70% zu verstehen ist. Auch andere Ausgestaltungen sind jedoch grundsätzlich möglich, beispielsweise Ausgestaltungen, bei welchen die flüssige Probe seitlich, beispielsweise parallel zu dem Schichtaufbau, in die Testchemie eingebracht wird.

Das Analysegerät kann insbesondere eingerichtet sein, um die Qualitätsmessung mindestens einmal vor der Analytmessung durchzuführen. So kann beispielsweise der Analytmessung mindestens eine Qualitätsmessung zeitlich vorgelagert sein. Wie oben ausgeführt, kann die Analytmessung von dem Ergebnis der Qualitätsmessung beeinflusst werden, beispielsweise indem die Analytmessung verhindert wird oder auf andere Weise beeinflusst wird, entsprechend dem Ergebnis der mindestens einen Qualitätsmessung.

Zur Durchführung der Analytmessung und/oder der Qualitätsmessung kann das Analysegerät entsprechende Vorrichtungen und/oder Elemente umfassen. So kann das Analysegerät insbesondere mindestens einen Analytdetektor umfassen, insbesondere mindestens einen optischen Analytdetektor. Unter einem optischen Analytdetektor ist dabei allgemein eine Vorrichtung zu verstehen, welche den mindestens einen Analytnachweis unter Verwendung einer oder mehrerer optischer Messtechniken durchführen kann. Insbesondere kann der optische Analytdetektor beispielsweise mindestens einen Photodetektor umfassen, beispielsweise mindestens ein fotoempfindliches Halbleiterbauelement wie beispielsweise eine Fotodiode und/oder mindestens eine CCD-Kamera. Optional kann der mindestens eine optische Analytdetektor darüber hinaus mindestens eine Lichtquelle umfassen, beispielsweise um die Testchemie mit mindestens einem Analyselicht zu bestrahlen, beispielsweise mindestens einem Anregungslicht und/oder mindestens einem Licht, was entsprechend der Reflektions- oder Remissionseigenschaften der Testchemie an der Testchemie reflektiert und/oder auf andere Weise durch die Testchemie beeinflusst wird. Wiederum alternativ oder zusätzlich kann die mindestens eine Lichtquelle mindestens ein Anregungslicht emittieren, welches die Testchemie zu mindestens einer Lumineszenz, insbesondere einer Fluoreszenz, anregen kann. Das Analysegerät kann allgemein eingerichtet sein, um bei der Analytmessung mittels des optischen Analytdetektors eine optische Erfassung der Eigenschaft der Testchemie durchzuführen, insbesondere eine Farbmessung und/oder eine Remissionsmessung und/oder eine Fluoreszenzmessung. Die mindestens eine optionale Lichtquelle des Analytdetektors kann eingerichtet sein, um eine oder mehrere Wellenlängen zu emittieren. Das von der Lichtquelle des Analytdetektors emittierte Licht wird im Folgenden auch als Analyselicht bezeichnet, wobei das von dem Analytdetektor, insbesondere mindestens einem Analytphotodetektor, erfasste Licht auch als Detektionslicht bezeichnet wird. Das Detektionslicht kann beispielsweise Analyselicht nach einer diffusen Streuung an dem Testelement oder Teilen desselben, beispielsweise der Testchemie, umfassen. Alternativ oder zusätzlich kann das Detektionslicht auch reflektiertes Analyselicht umfassen. Wiederum alternativ oder zusätzlich kann das Detektionslicht auch beispielsweise von der Testchemie emittiertes Licht umfassen, beispielsweise Fluoreszenz Licht, wobei die Lichtemission dieses emittierten Lichts durch das Analyselicht angeregt wird. Beispielsweise kann der Analytdetektor eingerichtet sein, um eine diffuse Reflexion, insbesondere eine Remission, an mindestens einer Schicht des Testelements zu erfassen, insbesondere an der Testchemie.

Wie oben ausgeführt, kann die Eigenlumineszenz insbesondere eine Eigenfluoreszenz der Testchemie umfassen. Allgemein kann die Eigenlumineszenz beispielsweise spektral aufgelöst und/oder integral über einen Wellenlängenbereich erfasst werden.

Das Analysegerät kann die erfasste Eigenlumineszenz oder einen zu dieser Eigenlumineszenz korrelierenden Wert, beispielsweise ein Detektorsignal, direkt als Qualität der Testchemie verwenden. Alternativ oder zusätzlich kann jedoch, wie oben ausgeführt und wie unten exemplarisch noch näher erläutert, die Qualität auch lediglich unter Verwendung der erfassten Eigenlumineszenz berechnet oder auf andere Weise bestimmt werden. So kann beispielsweise aus der Eigenlumineszenz zunächst, wie unten noch näher ausgeführt wird, auf eine Aktivität mindestens eines Enzyms der Testchemie und/oder mindestens eines Coenzyms der Testchemie geschlossen werden und/oder auf eine Aktivität einer anderen Substanz der Testchemie.

Das Analysegerät kann insbesondere eingerichtet sein, um auf eine Degradation des Testelements zu schließen, wenn die Eigenlumineszenz mindestens eine vorgegebene Schwelle überschreitet. So wurde, wie unten exemplarisch noch näher ausgeführt wird, bei gängigen Teststreifensystemen festgestellt, dass eine Eigenlumineszenz, insbesondere eine Eigenfluoreszenz, der Testchemie in vielen Fällen bei einer Alterung der Testelemente und insbesondere der Testchemie zunimmt. Insbesondere bei Testchemien, welche mindestens ein Enzym umfassen, beispielsweise Glucose-Oxidase und/oder Glucose-Dehydrogenase, lässt eine Zunahme der Eigenfluoreszenz der Testchemie auf eine Alterung der Testchemie schließen. Angesichts dieser empirischen Beobachtungen kann dahinstehen, ob diese Zunahme der Eigenfluoreszenz beispielsweise durch eine Eigenfluoreszenz von bei der Degradation entstehenden Abbauprodukten verursacht wird und/oder ob andere Prozesse eine Rolle spielen, wie beispielsweise eine Konformationsänderung der Testchemie während des Alterungsprozesses und dadurch beispielsweise eine reduzierte Fluoreszenzlöschung. Um die Zunahme der Eigenlumineszenz zu detektieren, können ein oder mehrere Schwellwerte als Schwelle vorgegeben werden. Beispielsweise kann dabei die Lumineszenz direkt mit dem mindestens einen Schwellwert verglichen werden, oder es können auch zunächst aus der mindestens einen Eigenlumineszenz weitere Kennwerte ermittelt werden, die dann mit dem mindestens einen Schwellwert verglichen werden.

Wie oben ausgeführt, kann die in der Qualitätsmessung ermittelte Qualität der Testchemie auf verschiedene Weisen genutzt werden. So kann beispielsweise, wenn festgestellt wird, dass die Qualität eine oder mehrere vorgegebene Bedingungen nicht erfüllt, beispielsweise wenn die Eigenlumineszenz einen oder mehrere Schwellwerte überschreitet, eine vorangehende Analytmessung verworfen oder eine nachfolgende Analytmessung verhindert werden, oder es kann eine Warnung an einen Benutzer ausgegeben werden. Alternativ oder zusätzlich kann die ermittelte Qualität auch bei der Auswertung der Analytmessung berücksichtigt werden, um beispielsweise eine Berechnung der Analytkonzentration in der Probe aus der bei der Analytmessung ermittelten veränderlichen Eigenschaft der Testchemie unter Berücksichtigung der in der Qualitätsmessung ermittelten Qualität durchzuführen. So kann das Analysegerät beispielsweise allgemein eingerichtet sein, um eine Berechnung einer Konzentration des Analyten in der Probe, beispielsweise angegeben in einer Masse des Analyten pro Volumen der Probe oder in einer Masse des Analyten pro Masse der Probe, unter Berücksichtigung der Qualität der Testchemie durchzuführen. Dies kann beispielsweise dadurch erfolgen, dass eine Korrektur der Analytmessung oder der aus der Analytmessung berechneten Konzentration des Analyten erfolgt, welche abhängig ist von der ermittelten Qualität. So können beispielsweise einfache Korrekturfaktoren verwendet werden, also beispielsweis lineare Korrekturen durchgeführt werden. Auch nicht-lineare Korrekturen sind jedoch grundsätzlich möglich. So können beispielsweise eine oder mehrere Korrekturfunktionen verwendet werden, welche beispielsweise in einem Datenspeicher des Analysegeräts hinterlegt sein können, welche die Berechnung der Analytkonzentration entsprechend der ermittelten Qualität durchführen. Diese Korrekturfunktionen können linear oder auch nicht-linear sein. Die Korrekturfunktionen können beispielsweise empirisch ermittelt werden. So kann beispielsweise eine bei der Qualitätsmessung eine Enzymaktivität ermittelt werden, und es kann beispielsweise aus einer Abnahme einer Enzymaktivität der Testchemie resultierende geringere Umsetzung des Analyten während eines enzymatischen Nachweises bei der Auswertung der Analytmessung berücksichtigt werden. Beispiele dieser Berücksichtigung werden unten noch näher erläutert.

Zur Durchführung der mindestens einen Qualitätsmessung kann das Analysegerät weiterhin mindestens einen optischen Qualitätsdetektor umfassen. Dieser optische Qualitätsdetektor kann ganz oder teilweise in den oben genannten optionalen Analytdetektor, insbesondere den optischen Analytdetektor, integriert sein oder kann auch ganz oder teilweise getrennt von dem mindestens einen optionalen Analytdetektor ausgestaltet sein. Dies bedeutet, dass der Qualitätsdetektor ganz oder teilweise bauteilgleich zu dem mindestens einen Analytdetektor ausgestaltet sein kann. Vorzugsweise ist der Qualitätsdetektor jedoch ganz oder teilweise von dem Analytdetektor verschieden ausgebildet und umfasst mindestens ein Element, beispielsweise mindestens eine Lichtquelle und/oder mindestens einen Detektor, insbesondere mindestens einen Photodetektor, welches nicht zugleich Bestandteil des Analytdetektors ist. So können der Analytdetektor und der Qualitätsdetektor unterschiedliche Lichtquellen und/oder unterschiedliche Photodetektoren umfassen. Alternativ oder zusätzlich können auch mindestens eine Lichtquelle und/oder mindestens ein Photodetektor sowohl in dem Qualitätsdetektor als auch in dem Analytdetektor eingesetzt werden. Beispielsweise kann mindestens eine Lichtquelle vorgesehen sein, welche sowohl Licht für den Analytdetektor als auch Licht für den Qualitätsdetektor zur Verfügung stellt, wobei dieses Zurverfügungstellen gleichzeitig oder auch zu unterschiedlichen Zeiten erfolgen. Das für den Analytdetektor zur Verfügung gestellte Licht kann spektral verschieden sein von dem Licht, welches für den Qualitätsdetektor zur Verfügung gestellt wird oder auch spektral identisch. Beispielsweise kann der Analytdetektor mindestens eine Analytlichtquelle aufweisen, welche zur Erzeugung von Analyselicht eingesetzt wird. Beispielsweise kann dies Analyselicht mit einer Wellenlänge von 360 nm sein. Dieselbe Analytlichtquelle kann, gleichzeitig oder zeitversetzt, auch als Bestandteil des Qualitätsdetektors eingesetzt werden und kann beispielsweise Anregungslicht oder anderes Licht für eine Qualitätsmessung bereitstellen. Dieses Anregungslicht kann beispielsweise dieselbe Wellenlänge oder dieselben spektralen Eigenschaften aufweisen wie das Analysegerät. Alternativ kann dieses Anregungslicht auch unterschiedliche spektrale Eigenschaften aufweisen, beispielsweise eine unterschiedliche Wellenlänge.

So kann der mindestens eine optische Qualitätsdetektor beispielsweise mindestens eine Lichtquelle umfassen, welche eingerichtet ist, um die Testchemie vollständig oder teilweise mit mindestens einem Anregungslicht zu bestrahlen, insbesondere Licht im ultravioletten und/oder sichtbaren Spektralbereich. Beispielsweise kann mindestens eine Anregungslichtquelle vorgesehen sein, welche eingerichtet ist, um die Testchemie mit ultraviolettem Licht einer Wellenlänge von 340 nm bis 380 nm, beispielsweise 360 nm, zu bestrahlen. Weiterhin kann der optische Qualitätsdetektor mindestens ein fotoempfindliches Element aufweisen, welches eingerichtet ist, um mindestens eine Lumineszenz der Testchemie qualitativ oder vorzugsweise quantitativ zu erfassen. Beispielsweise kann zu diesem Zweck mindestens eine Fotodiode, mindestens eine CCD-Kamera, mindestens ein Photodetektor oder mindestens eine andere Art eines fotoempfindlichen Elements vorgesehen sein. Weiterhin kann der optische Qualitätsdetektor zusätzliche optische Elemente umfassen, wie beispielsweise einen oder mehrere Filter zur Filterung des Anregungslichts und/oder zur Filterung der Lumineszenz.

Insbesondere kann das Analysegerät eingerichtet sein, um mittels des optischen Qualitätsdetektors die Eigenlumineszenz in mindestens zwei verschiedenen Wellenlängenbereichen zu erfassen. So kann beispielsweise eine erste Eigenlumineszenz in einem ersten Wellenlängenintervall und mindestens eine zweite Eigenlumineszenz in mindestens einem zweiten Wellenlängenintervall erfasst werden. Die mindestens eine erste Eigenlumineszenz und die mindestens eine zweite Eigenlumineszenz, wobei weitere Eigenlumineszenzen vorgesehen sein können, können beispielsweise integral über die relevanten Wellenlängenintervalle erfasst werden. Alternativ kann auch eine spektrale Auflösung der Erfassung der Eigenlumineszenzen erfolgen. Insbesondere kann der optische Qualitätsdetektor eingerichtet sein, um mindestens eine erste Eigenlumineszenz oder mindestens ein erstes Eigenlumineszenz-Spektrum in einem ersten Wellenlängenintervall und mindestens eine zweite Eigenlumineszenz oder mindestens ein zweites Eigenlumineszenz-Spektrum in mindestens einem zweiten Wellenlängenintervall zu erfassen. Zur Erfassung der mindestens zwei Eigenlumineszenzen können unterschiedliche fotoempfindliche Elemente, beispielsweise unterschiedliche Photodetektoren und/oder unterschiedliche Fotodioden, vorgesehen sein. Alternativ können die unterschiedlichen Lumineszenzen auch mit ein und demselben Detektor erfasst werden, beispielsweise indem zeitversetzt zunächst eine Erfassung einer durch einen ersten optischen Filter gefilterten Lumineszenz und anschließend einer durch einen zweiten optischen Filter gefilterten Lumineszenz erfolgt. Alternativ zur Verwendung von Filtern unterschiedlicher spektraler Eigenschaften können auch beispielsweise foto-empfindliche Elemente mit unterschiedlichen spektralen Eigenschaften eingesetzt werden. Allgemein kann der optische Qualitätsdetektor vorzugsweise mindestens einen ersten Lumineszenz-Detektor zur Erfassung der ersten Eigenlumineszenz, sowie optional mindestens einen ersten optischen Filter, und mindestens einen zweiten Lumineszenz-Detektor, ggf. mit mindestens einem zweiten optischen Filter, zur Erfassung der zweiten Eigenlumineszenz aufweisen.

Die mindestens zwei Eigenlumineszenzen, welche optional erfasst werden können, können auf verschiedene Weise genutzt werden, um die Qualität zu bestimmen. So kann das Analysegerät insbesondere eingerichtet sein, um aus der Eigenlumineszenz in den mindestens zwei verschiedenen Wellenlängenbereichen, beispielsweise aus der ersten Eigenlumineszenz und der zweiten Eigenlumineszenz, mindestens eine die Qualität charakterisierende Qualitätskennzahl zu berechnen. Diese Qualitätskennzahl kann beispielsweise durch eine einfache Quotientenbildung aus der ersten Eigenlumineszenz und der zweiten Eigenlumineszenz berechnet werden. Alternativ oder zusätzlich kann auch beispielsweise eine Linearkombination der Eigenlumineszenzen gebildet werden, beispielsweise gemäß einer vorgegebenen Vorschrift. Auch andere Funktionen zur Berechnung der Qualitätskennzahl aus den mindestens zwei Eigenlumineszenzen sind einsetzbar. Beispiele werden unten noch näher berechnet.

Insbesondere für derzeit verwendete enzymatische Nachweise, jedoch auf für andere Arten von Testchemien hat es sich gezeigt, dass eine Trennung der Eigenlumineszenzen in Eigenlumineszenzen im ultravioletten Spektralbereich und im sichtbaren Spektralbereich günstig sein kann. So kann die erste Eigenlumineszenz beispielsweise integral in einem ersten Wellenlängenbereich von 380 nm bis 420 nm erfasst werden, und die zweite Eigenlumineszenz integral in einem zweiten Wellenlängenbereich von mindestens 420 nm oder von über 420 nm, beispielsweise in einem Wellenlängenbereich von 420 nm bis 650 nm.

Der Qualitätsdetektor kann, wie oben beschrieben, insbesondere mindestens eine Anregungslichtquelle aufweisen. Diese mindestens eine Anregungslichtquelle kann beispielsweise eine Halbleiterlichtquelle umfassen. Auch andere Lichtquellen sind jedoch grundsätzlich einsetzbar, wie beispielsweise Glühlampen, Gasentladungslampen, Laserlichtquellen oder andere Arten von Anregungslichtquellen. Auch eine Kombination mehrerer Anregungslichtquellen unterschiedlicher oder gleicher Art ist grundsätzlich denkbar. Insbesondere kann die Anregungslichtquelle eingerichtet sein, um die Testchemie mit einem Anregungslicht mit einer Anregungswellenlänge von 340 nm bis 380 nm zu bestrahlen, insbesondere mit einem Anregungslicht von 360 nm.

Das Analysegerät kann weiterhin mindestens eine Auswertevorrichtung umfassen. Diese Auswertevorrichtung kann beispielsweise mindestens eine Datenverarbeitungsvorrichtung umfassen, beispielsweise mindestens einen Mikrocomputer. Weiterhin kann die Auswertevorrichtung beispielsweise einen oder mehrere flüchtige und/oder nicht-flüchtige Datenspeicher umfassen. Die Auswertevorrichtung kann beispielsweise programmtechnisch eingerichtet sein, beispielsweise um aus der während der Analytmessung erfassten veränderlichen Eigenschaft auf die Anwesenheit des Analyten in der Probe und/oder auf eine Konzentration des Analyten in der Probe zu schließen. Dementsprechend kann die Auswertevorrichtung beispielsweise eine oder mehrere Auswertefunktionen umfassen, welche beispielsweise programmtechnisch implementiert sein können, und über welche aus der erfassten Eigenschaft der Testchemie, beispielsweise einer spektralen Eigenschaft, einem Farbumschlag, einer Remission oder anderen Eigenschaften, auf die Analytkonzentration geschlossen werden kann. Weiterhin kann die Auswertevorrichtung auch eingerichtet sein, um die oben beschriebene Berücksichtigung der Qualität der Testchemie bei der Berechnung der Konzentration des Analyten in der Probe aus der erfassten Eigenschaft vorzunehmen. So kann beispielsweise die mindestens eine Korrektur, welche die Qualität der Testchemie berücksichtigt, in der Auswertevorrichtung implementiert werden, beispielsweise indem ein oder mehrere Korrekturfaktoren und/oder ein oder mehrere Korrekturfunktionen in diese Auswertevorrichtung hinterlegt sind. Beispielsweise kann die Auswertevorrichtung auch eine elektronische Tabelle umfassen, in welcher ein oder mehrere Korrekturfunktionen und/oder ein oder mehrere Korrekturfaktoren zur Korrektur der Analytkonzentration entsprechend der erfassten Qualität hinterlegt sein können. Weiterhin kann die Auswertevorrichtung auch eingerichtet sein, um die Qualität mit mindestens einer Bedingung zu vergleichen, insbesondere um die Qualität mit mindestens einem Schwellwert zu vergleichen, beispielsweise gemäß der obigen Beschreibung. Wie oben ausgeführt, können von dem Ergebnis dieses Vergleichs bestimmte Handlungen abhängig gemacht werden, beispielsweise die Auswertung einer bereits erfolgten Analytmessung und/oder die Freigabe einer noch zu erfolgenden Analytmessung und/oder die Ausgabe einer Warnung oder einer Information an einen Benutzer und/oder ein weiteres Gerät. Das Analysegerät ist, beispielsweise durch eine entsprechende programmtechnische Einrichtung der Auswertevorrichtung, eingerichtet, um entsprechend der erfassten Qualität mindestens eine Aktion durchzuführen. Die Aktion ist ausgewählt aus der Gruppe bestehend aus: einer Ausgabe eines Hinweises an einen Benutzer; insbesondere eines Warnhinweises; einer Weitergabe mindestens einer Information über die Qualität an mindestens ein weiteres Gerät, beispielsweise einen externen Computer und/oder einen Arztcomputer; einer Abspeicherung mindestens einer Information über die Qualität in mindestens einem Datenspeicher; einer Verhinderung oder Ermöglichung der Analytmessung, also beispielsweise einer noch nicht erfolgten Analytmessung; einer Berücksichtigung oder Nichtberücksichtigung einer bereits erfolgten Analytmessung.

Das Analysegerät umfasst weiterhin mindestens ein Testelement, beispielsweise mindestens ein Testelement der oben beschriebenen Art. Das Testelement umfasst mindestens eine Testchemie, beispielsweise in Form eines oder mehrerer der oben beschriebenen Testfelder. Bezüglich des möglichen Aufbaus des mindestens einen Testelements kann auf die obige Beschreibung verwiesen werden. Die Testchemie ist dabei eingerichtet, um durch Anwesenheit des Analyten mindestens eine Eigenschaft zu ändern. Das Testelement kann beispielsweise fest in dem Analysegerät integriert sein, kann jedoch auch grundsätzlich herausnehmbar in dem Analysegerät aufgenommen sein, beispielsweise in Form eines oder mehrerer Testelemente, welche auch magaziniert in dem Analysegerät vorliegen können.

Die Testchemie kann insbesondere mindestens ein Enzym umfassen. Diese mindestens eine Enzym kann insbesondere ausgewählt sein aus der Gruppe bestehend aus einer Oxidase, beispielsweise eine Glucoseoxidase, und einer Dehydrogenase, beispielsweise einer Glucose-Dehydrogenase. Unter Dehydrogenasen können im Rahmen der vorliegenden Erfindung insbesondere Polypeptide verstanden werden, welche in der Lage sind, eine Reaktion eines Substrats durch Transfer von Hydriden (H⁻) als Redoxäquivalente auf ein Akzeptormolekül zu katalysieren, vorzugsweise auf einen Redox-Cofaktor. Der Begriff Redox-Cofaktor bezieht sich dabei allgemein auf ein Molekül, welches als Akzeptor für enzymatisch transferierte Redoxäquivalente dienen kann, und insbesondere auf Hydride (H⁻). Dehydrogenasen können insbesondere von einem Redox-Cofaktor abhängen, welcher bisweilen auch als Coenzym bezeichnet wird. Insbesondere können eine oder mehrere Dehydrogenasen verwendet werden, welche von mindestens einem Coenzym abhängen, ausgewählt aus der Gruppe bestehend aus: Pyrrolo-Quinolin-Quinin (PQQ); Nicotinamid-Adenin-Dinucleotid (NAD) oder einem Derivat davon; einem Flavin, insbesondere Flavin-Adenin-Dinucleotid (FAD) oder Flavin-Mononucleotid (FMN).

Insbesondere kann das Enzym ausgewählt sein aus der Gruppe bestehend aus: Glucose-Dehydrogenase (E.C.1.1.1.47); Lactat-Dehydrogenase (E.C.1.1.1.27, 1.1.1.28); Malat-Dehydrogenase (E.C.1.1.1.37); Glycerin-Dehydrogenase (E.C.1.1.1.6); Alkohol-Dehydrogenase (E.C.1.1.1.1); alpha-Hydroxybutyrat-Dehydrogenase; Sorbitol-Dehydrogenase; Aminosäure-Dehydrogenase, insbesondere L-Aminosäure-Dehydrogenase (E.C.1.4.1.5); Glucoseoxidase (E.C.1.1.3.4); Cholesterinoxidase (E.C.1.1.3.6); Aminotransferasen, insbesondere Aspartat oder Alanin Aminotransferase; 5'-Nukleotidase; Creatin-Kinase; Glucose 6-Phosphat-Dehydrogenase (EC 1.1.1.49); NAD-abhängiger Cholesterol-Dehydrogenase (EC 1.1.1.62); FAD-abhängiger Glucose-Dehydrogenase (EC 1.1.99.10); PQQ-abhängiger Glucose-Dehydrogenase (EC 1.1.5.2).

Weiterhin kann die Testchemie weitere Substanzen umfassen, wie beispielsweise ein oder mehrere Coenzyme und/oder einen oder mehrere Mediatoren und/oder einen oder mehrere Farbstoffe. Besonders bevorzugt umfasst die Testchemie eine Kombination aus Glucose-Dehydrogenase und cNAD sowie optional mindestens einem Farbstoff. Auch andere Ausgestaltungen der Testchemie oder andere Kombinationen sind jedoch möglich. Beispielsweise kann bezüglich möglicher Testchemien auf sämtliche der oben genannten Dokumente des Standes der Technik verwiesen werden. Diese Testchemien können grundsätzlich auch im Rahmen der vorliegenden Erfindung zum Einsatz kommen. Insbesondere kann bezüglich einer möglichen Testchemie sowie der möglichen Herstellung eines Testelements auf die WO 2010/094426 verwiesen werden. Auch andere Testchemien, beispielsweise gemäß den übrigen oben angegebenen Druckschriften zum Stand der Technik, sind jedoch grundsätzlich im Rahme der vorliegenden Erfindung einsetzbar.

Wird eine Testchemie verwendet, welche mindestens ein Enzym umfasst, so kann die Qualität insbesondere mindestens eine Information über eine Aktivität des Enzyms umfassen. Die Aktivität eines Enzyms stellt ein Maß dafür dar, wie schnell ein durch das Enzym umgesetztes Edukt zu Produkten umgesetzt wird. Die Aktivität kann sich dabei beispielsweise auf die gesamte Testchemie, einen Teil derselben oder lediglich auf die Enzyme beziehen. So kann beispielsweise die Geschwindigkeit einer Umsetzung des nachzuweisenden Analyten bestimmt und daraus beispielsweise eine Geschwindigkeitskonstante ermittelt werden. Beispiele einer Bestimmung einer Enzymaktivität auf herkömmliche Weise, welche einzeln oder in beliebiger Kombination auch im Rahmen der vorliegenden Erfindung zur Bestimmung der Enzymaktivität herangezogen werden können, werden nachfolgend noch näher beschrieben.

Die Testchemie kann insbesondere zumindest weitgehend stabil gegenüber Umwelteinflüssen, insbesondere gegenüber Feuchtigkeit, sein. Die Testchemie kann insbesondere als Trockenchemie vorliegen, insbesondere auf einem Teststreifen. Dabei wird unter einer im Wesentlichen gegenüber Umwelteinflüssen stabilen Testchemie eine Testchemie verstanden, die gegenüber Luftfeuchtigkeit und vorteilhafterweise ebenfalls gegenüber einer erhöhten Temperatur und/oder gegenüber einer Bestrahlung mit ultraviolettem Licht und/oder gegenüber Sterilisationsverfahren, insbesondere Sterilisationsverfahren unter Verwendung ionisierender Strahlung, stabil ist. Insbesondere kann eine Bestrahlung mit ultraviolettem Licht beispielsweise auftreten, wenn eine Analytmessung beispielsweise in den Bergen oder am Strand durchgeführt wird. Allgemein kann die Testchemie insbesondere als stabil bezeichnet werden, wenn bei einer Lagerung bei 32 °C, einer relativen Luftfeuchtigkeit von 85% bei Normaldruck über eine Zeitdauer von drei Wochen die Aktivität, beispielsweise die Enzymaktivität der Testchemie des analytischen Hilfsmittels, um weniger als 50%, vorzugsweise um weniger als 30% und besonders bevorzugt um weniger als 20% abnimmt. Die Aktivität kann dabei grundsätzlich mittels eines beliebigen, aus dem Stand der Technik bekannten Verfahrens bestimmt werden, da im Rahmen der angegebenen Definition lediglich ein Verhältnis der Abnahme der mit diesem Verfahren gemessenen Aktivität zu einer mit diesem Verfahren gemessenen Aktivität vor der Lagerung bzw. unmittelbar nach der Herstellung des analytischen Hilfsmittels von Relevanz ist. Die Aktivität kann sich dabei insbesondere auf eine Enzymaktivität einer Trockenchemie beziehen, insbesondere in einem Teststreifen. Beispielsweise sind Verfahren bekannt, die zur Messung der Enzymaktivität das Enzym aus der Testchemie bzw. dem Teststreifen extrahieren und anschließend beispielsweise mittels einer Ultraviolett-Absorption die Aktivität bestimmen. Diesbezüglich kann beispielsweise auf H. U. Bergmeyer: Methoden der enzymatischen Analyse, Verlag Chemie, 2. Auflage 1970, S. 417 oder auf Banauch et al.: A glucose dehydrogenase for the determination of glucose concentrations in body fluids, Z. Klin. Chem. Klin. Biochem. 1975 Mar; 13(3):101-7 verwiesen werden.

Beispielsweise kann für den Test ein Teststreifen oder eine andere Art von Testelement mit der Testchemie hergestellt werden, mit einem üblichen Verfahren die Enzymaktivität eines Enzyms der Testchemie gemessen werden, dann die oben beschriebene Lagerung durchgeführt werden und anschließend erneut dasselbe Verfahren zur Messung der Enzymaktivität durchgeführt werden. Diese Prozedur wird üblicherweise mit einem repräsentativen Kollektiv an Testelementen bzw. Testchemien durchgeführt. Alternativ oder zusätzlich zu einer Stabilität gegenüber Umwelteinflüssen in Form von Luftfeuchtigkeit kann vorzugsweise auch eine hohe Stabilität der Testchemie gegenüber Umwelteinflüssen in Form von üblicherweise für eine Sterilisierung der analytischen Hilfsmittel und/oder der analytischen Magazine insgesamt verwendeten Strahlung gegeben sein, beispielsweise einer Gammastrahlung und/oder Betastrahlung und/oder einer anderen Art von ionisierender Strahlung.

Als Beispiel einer derartigen, gegenüber Umwelteinflüssen stabilen Testchemie kann auf die oben bereits zitierte WO 2007/012494 A1 verwiesen werden. Die dort dargestellte Testchemie ist auch im Rahmen der vorliegenden Erfindung einsetzbar, allein oder auch in Kombination mit einer oder mehreren anderen Testchemien. Alternativ oder zusätzlich kann die Testchemie auch ausgestaltet sein wie in der europäischen Patentanmeldung mit der Nummer EP 2 093 284 A1 oder in der internationalen Patentanmeldung WO 2009/103540 A1 aus dem Hause der Anmelderin der vorliegenden Patentanmeldung beschrieben.

So kann die Testchemie beispielsweise ein Enzym und ein stabiles Coenzym enthalten, welche gemeinsamen gelagert sind. Überraschenderweise wurde gefunden, dass mit Hilfe eines stabilen Coenzyms eine Langzeitstabilisierung von mehreren Wochen bzw. Monaten bei hoher relativer Feuchte oder sogar in flüssiger Phase und bei erhöhten Temperaturen möglich ist. Diese Erkenntnis ist überraschend, da bekannt ist, dass Enzyme in Gegenwart von nativem Coenzym zwar eine erhöhte Kurzzeitstabilität für einige Stunden besitzen, aber eine geringere Haltbarkeit über einen längeren Zeitraum aufweisen. Gegenüber diesen Erkenntnissen gegenüber dem Stand der Technik war es überraschend, dass ein Enzym in Gegenwart eines stabilen Coenzyms eine deutlich erhöhte Langzeitstabilität als ein Enzym in Gegenwart eines nativen Coenzyms besitzt, insbesondere da die stabilen Coenzyme eine niedrigere Bindungskonstante mit dem Enzym als das native Coenzym besitzen.

Die Testchemie kann, wie oben ausgeführt, insbesondere mindestens ein Enzym umfassen, vorzugsweise ein stabilisiertes Enzym. Das Enzym, insbesondere das stabilisierte Enzym, kann insbesondere ein Coenzym-abhängiges Enzym sein. Geeignete Enzyme sind z.B. Dehydrogenasen, ausgewählt aus einer Glucose-Dehydrogenase (E.C.1.1.1.47), Lactat-Dehydrogenase (E.C.1.1.1.27, 1.1.1.28), Malat-Dehydrogenase (E.C.1.1.1.37), Glycerin-Dehydrogenase (E.C.1.1.1.6), Alkohol-Dehydrogenase (E.C.1.1.1.1), alpha-Hydroxybutyrat-Dehydrogenase, Sorbitol-Dehydrogenase oder Aminosäure-Dehydrogenase, z. B. L-Aminosäure-Dehydrogenase (E.C.1.4.1.5). Weitere geeignete Enzyme sind Oxidasen, wie etwa Glucoseoxidase (E.C.1.1.3.4) oder Cholesterinoxidase (E.C.1.1.3.6) bzw. Aminotransferasen, wie z.B. Aspartat oder Alanin Aminotransferase, 5'-Nukleotidase oder Creatin-Kinase; Glucose 6-Phosphat-Dehydrogenase (EC 1.1.1.49); NAD-abhängiger Cholesterol-Dehydrogenase (EC 1.1.1.62); FAD-abhängiger Glucose-Dehydrogenase (EC 1.1.99.10); PQQ-abhängiger Glucose-Dehydrogenase (EC 1.1.5.2). Vorzugsweise ist das Enzym Glucose-Dehydrogenase. Auch Mutanten der zuvor genannten Enzyme sind einsetzbar und kommen insbesondere als stabilisierte Enzyme in Betracht.

Als besonders bevorzugt hat sich der Einsatz einer mutierten Glucose-Dehydrogenase erwiesen. Der Begriff "Mutante", wie er im Rahmen der vorliegenden Anmeldung verwendet wird, bezeichnet eine genetisch veränderte Variante eines nativen Enzyms. Die genetisch veränderte Variante des nativen Enzyms kann sich in mindestens einer Aminosäure vom Wildtyp-Enzym unterscheiden. Die genetisch veränderte Variante des nativen Enzyms kann im Vergleich zu dem Wildtyp-Enzym eine gleiche Anzahl an Aminosäuren oder auch eine unterschiedliche Anzahl an Aminosäuren aufweisen. Insbesondere kann die Mutante auch mindestens eine Deletion, mindestens eine Substitution und/oder mindestens eine Insertion umfassen. Insbesondere kann unter einer Mutante eine genetisch veränderte Variante eines nativen Enzyms verstanden werden, welche zu dem Wildtyp-Enzym eine Sequenzhomologie von mindestens 80 %, vorzugsweise von mindestens 90 % und besonders bevorzugt von mindestens 95 % aufweist. Bevorzugt ist unter Sequenzhomologie Sequenzidentität zu verstehen. Diese kann besonders in einem Vergleichsfenster bestimmt werden, das sich über die gesamte Länge der zu vergleichenden, optimal zueinander angeordneten Aminosäuresequenzen erstreckt. Ebenso bevorzugt kann die Berechnung auch nur in einem Vergleichsfenster erfolgen, das sich über einen Teilbereich der zu vergleichenden, optimal zueinander angeordneten Aminosäuresequenzen erstreckt. Dieser Teilbereich soll besonders bevorzugt mindesten die Hälfte der Gesamtzahl an Aminosäuren der Längen der beiden Aminosäuresequenzen umfassen. Zur Bestimmung der Sequenzidentität (in Prozent) wird die Zahl der identischen Aminosäuren im Vergleichsfenster durch die Gesamtzahl der Aminosäuren der beiden zu vergleichenden Sequenzen im Vergleichsfenster geteilt und mit 100 multipliziert. Zwei Aminosäuresequenzen können optimal zueinander angeordnet werden mittels im Stand der Technik bekannter Algorithmen für Aminosäuresequenzvergleiche. Besonders bevorzugt kann der BLASTP Algorithmus eingesetzt mit den standardmäßig vorgegebenen Parametern. Bevorzugt weist die Mutante des Enzyms noch im Wesentlichen dieselbe Aktivität wie das native Enzym auf Mutanten der zuvor genannten nativen Enzyme sollten bevorzugt zudem von Nukleinsäuremolekülen kodiert werden, die in der Lage sind unter stringenten Hybridisierungsbedingungen mit den Nukleinsäuremolekülen zu hybridisieren, die die oben genannten nativen Enzyme kodieren. Unter stringenten Hybridisierungsbedingungen ist bevorzugt eine Hybridisierung zu verstehen, bei der die zu hybridisierenden Nukleinsäuren in Church Puffer (0,5 M NaPO₄ (pH 7,15), 7% SDS; 1mM EDTA) für 12 Stunden bei 65°C inkubiert und anschließend zweimal für ca. 30 min in Waschpuffer (40mM NaPO₄ (pH 7,15), 1% SDS; 1mM EDTA) gewaschen werden. Eine der zu hybridisierenden Nukleinsäuren ist hierbei immobilisiert, die andere mit einer nachweisbaren Markierung versehen. Sofern die Nukleinsäuren miteinander hybridisieren, kann diese Hybridisierung über den nachweisbare Markierung an der immobilisierten Nukleinsäure nachgewiesen werden. Weitere Einzelheiten zur Durchführung von Hybridisierungsreaktionen sind im Stand der Technik bekannt. Die Einführung der einen oder mehreren Mutationen kann ortsspezifisch oder nicht-ortsspezifisch, bevorzugt ortsspezifisch unter Verwendung von im Fachbereich bekannten rekombinanten Methoden, erfolgen, wobei entsprechend den jeweiligen Anforderungen und Bedingungen mindestens ein Aminosäureaustausch innerhalb der Aminosäuresequenz des nativen Enzyms resultiert. Besonders bevorzugt weist die Mutante eine gegenüber dem Wildtyp-Enzym erhöhte thermische oder hydrolytische Stabilität auf.

Die mutierte Glucose-Dehydrogenase kann die gegenüber der korrespondierenden Wildtyp-Glucose-Dehydrogenase veränderte(n) Aminosäure(n) grundsätzlich an einer beliebigen Position ihrer Aminosäuresequenz enthalten. Vorzugsweise umfasst die mutierte Glucose-Dehydrogenase eine Mutation an mindestens einer der Positionen 96, 170 und 252 der Aminosäuresequenz der Wildtyp-Glucose-Dehydrogenase, wobei Mutanten mit Mutationen an Position 96 und Position 170 bzw. Mutationen an Position 170 und Position 252 besonders bevorzugt sind. Als vorteilhaft hat es sich erwiesen, wenn die mutierte Glucose-Dehydrogenase neben diesen Mutationen keine weiteren Mutationen enthält.

Die Mutation an den Positionen 96, 170 und 252 kann grundsätzlich einen beliebigen Aminosäureaustausch umfassen, welcher zu einer Stabilisierung, z.B. einer Erhöhung der thermischen oder hydrolytischen Stabilität, des Wildtyp-Enzyms führt. Vorzugsweise umfasst die Mutation an Position 96 einen Aminosäureaustausch von Glutaminsäure gegen Glycin, während in Bezug auf Position 170 ein Aminosäureaustausch von Glutaminsäure gegen Arginin oder Lysin, insbesondere ein Aminosäureaustausch von Glutaminsäure gegen Lysin, bevorzugt ist. Was die Mutation an Position 252 anbetrifft, so umfasst diese bevorzugt einen Aminosäureaustausch von Lysin gegen Leucin.

Die mutierte Glucose-Dehydrogenase kann durch Mutation einer aus einer beliebigen biologischen Quelle stammenden Wildtyp-Glucose-Dehydrogenase erhalten werden, wobei der Begriff "biologische Quelle" im Sinne dieser Erfindung sowohl Prokaryoten, wie beispielsweise Bakterien, als auch Eukaryoten, wie beispielsweise Säuger und andere Tiere, umfasst. Vorzugsweise entstammt die Wildtyp-Glucose-Dehydrogenase einem Bakterium, wobei es sich besonders bevorzugt um eine Glucose-Dehydrogenase aus Bacillus megaterium, Bacillus subtilis oder Bacillus thuringiensis, insbesondere aus Bacillus subtilis, handelt.

In einer besonders bevorzugten Ausführungsform handelt es sich bei der mutierten Glucose-Dehydrogenase um eine durch Mutation von Wildtyp-Glucose-Dehydrogenase aus Bacillus subtilis erhaltene Glucose-Dehydrogenase, welche die in SEQ ID NO: 1 (GlucDH_E96G_E170K) oder die in SEQ ID NO: 2 (GlucDH_E170K_K252L) dargestellte Aminosäuresequenz besitzt.

Die Testchemie kann weiterhin mindestens ein Coenzym umfassen, insbesondere ein stabiles Coenzym, Das Coenzym, insbesondere das stabile Coenzym, ist vorzugsweise ein gegenüber dem nativen Coenzym chemisch verändertes Coenzym, welches eine im Vergleich zum nativen Coenyzm höhere Stabilität, (z.B. hydrolytische Stabilität) aufweist. Vorzugsweise ist das stabile Coenyzm unter Testbedingungen gegenüber Hydrolyse stabil. Im Vergleich zum nativen Coenzym kann das stabile Coenzym eine verringerte Bindungskonstante für das Enzym aufweisen, beispielsweise eine um den Faktor von 2 oder mehr verringerte Bindungskonstante.

Bevorzugte Beispiele für stabile Coenzyme sind stabile Derivate von Nicotinamid-Adenin-Dinukleotid (NAD/NADH) oder Nicotinamid-Adenin-Dinukleotidphosphat (NADP/NADPH), oder verkürzte NAD-Derivate, z.B. ohne AMP-Teil bzw. mit nicht nukleosidischen Resten, z.B. hydrophoben Resten. Ebenfalls bevorzugt ist als stabiles Coenzym im Sinne der vorliegenden Erfindung die Verbindung der Formel (I).

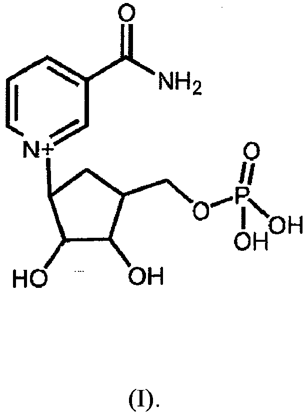

Bevorzugte stabile Derivate von NAD/NADH bzw. NADP/NADPH sind in den zuvor genannten Referenzen beschrieben, auf deren Offenbarung hiermit ausdrücklich Bezug genommen wird. Besonders bevorzugte stabilisierte Coenzyme sind in WO 2007/012494 bzw. US 11/460,366 beschrieben, auf deren Offenbarung hiermit ausdrücklich Bezug genommen wird. Das stabile Coenzym wird besonders bevorzugt aus Verbindungen mit der allgemeinen Formel (II) ausgewählt: 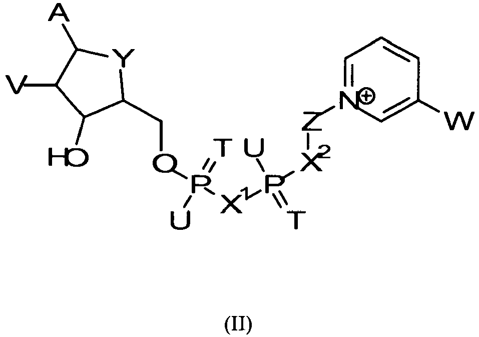 mit
- A =: Adenin oder ein Analog davon,
- T =: jeweils unabhängig O, S,
- U =: jeweils unabhängig OH, SH, BH₃⁻, BCNH₂⁻,
- V =: jeweils unabhängig OH oder eine Phosphatgruppe, oder zwei Gruppen, die eine cyclische Phosphatgruppe bilden;
- W =: COOR, CON(R)₂, COR, CSN(R)₂ mit R = jeweils unabhängig H oder C₁-C₂-Alkyl,
- X¹, X² =: jeweils unabhängig O, CH₂, CHCH₃, C(CH₃)₂, NH, NCH₃,
- Y =: NH, S, O, CH₂,
- Z =: ein linearer oder cyclischer organischer Rest ist,
mit der Maßgabe, dass Z und der Pyridin-Rest nicht durch eine glycosidische Verbindung verknüpft sind, oder ein Salz oder gegebenenfalls eine reduzierte Form davon.

In den Verbindungen der Formel (II) ist Z vorzugsweise ein linearer Rest mit 4-6 C-Atomen, vorzugsweise 4 C-Atomen, worin 1 oder 2 C-Atome gegebenenfalls durch ein oder mehrere Heteroatome ausgewählt aus O, S und N ersetzt sind, oder ein Rest umfassend eine cyclische Gruppe mit 5 oder 6 C-Atomen, die gegebenenfalls ein Heteroatom ausgewählt aus O, S und N sowie gegebenenfalls einen oder mehrere Substituenten enthält, und einen Rest CR⁴₂, wobei CR⁴₂ an die cyclische Gruppe und an X² gebunden ist, mit R⁴ = jeweils unabhängig H, F, Cl, CH₃.

Besonders bevorzugt ist Z ein gesättigter oder ungesättigter carbocyclischer oder heterocyclischer Fünfring, insbesondere eine Verbindung der allgemeinen Formel (III) 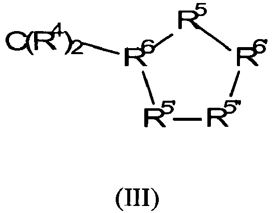 wobei zwischen R⁵' und R⁵" eine Einfach- oder Doppelbindung vorliegen kann, mit
R⁴ = jeweils unabhängig H, F, Cl, CH₃,
R⁵ =CR⁴₂,
wobei R⁵' = O, S, NH, NC₁-C₂-Alkyl, CR⁴₂, CHOH, CHOCH₃, und
R⁵" = CR⁴₂, CHOH, CHOCH₃, wenn zwischen R⁵' und R⁵" eine Einfachbindung vorliegt, und
wobei R⁵'=R⁵"=CR⁴, wenn zwischen R⁵' und R⁵" eine Doppelbindung vorliegt, und
R⁶, R⁶' = jeweils unabhängig CH oder CCH₃.

In einer bevorzugten Ausführungsform enthalten die Verbindungen Adenin oder Adenin-Analoga, wie z.B. C₈- und N₆-substituiertes Adenin, Deaza-Varianten wie 7-Deaza, Azavarianten wie 8-Aza oder Kombinationen wie 7-Deaza oder 8-Aza oder carbocyclische Analoga, wie Formycin, wobei die 7-Deazavarianten in der 7-Position mit Halogen, C₁-C₆-Alkinyl, -Alkenyl oder -Alkyl substituiert sein können.

In einer weiteren bevorzugten Ausführungsform enthalten die Verbindungen Adenosin-Analoga, welche statt Ribose z.B. 2-Methoxy-deoxyribose, 2'-Fluoro-deoxyribose, Hexitol, Altritol bzw. polycyclische Analoga, wie Bicyclo-, LNA- und Tricyclo-Zucker enthalten.

Insbesondere können in den Verbindungen der Formel (II) auch (Di-)-Phosphatsauerstoffe isotronisch ersetzt sein, wie z.B. O⁻ durch S⁻ bzw. BH₃⁻, O durch NH, NCH₃ bzw. CH₂ und =O durch =S.

In den erfindungsgemäßen Verbindungen der Formel (II) ist W vorzugsweise CONH₂ oder COCH₃.

In den Gruppen der Formel (III) ist R⁵ vorzugsweise CH₂. Weiterhin ist bevorzugt, dass R⁵' ausgewählt ist aus CH₂, CHOH und NH. In einer besonders bevorzugten Ausführungsform sind R⁵' und R⁵" jeweils CHOH. In noch einer weiteren bevorzugten Ausführungsform ist R⁵' NH und R⁵" CH₂.

In der am stärksten bevorzugten Ausführungsform handelt es sich bei dem stabilen Coenzym um carbaNAD (cNAD).

Die bevorzugte Testchemie ist insbesondere derart ausgestaltet, dass das bevorzugt enthaltene mindestens eine Enzym Langzeit-stabilisiert ist. Dies bedeutet, dass man das mit einem stabilen Coenzym stabilisierte Enzym, z.B. als Trockensubstanz, beispielsweise über eine Dauer von mindestens zwei Wochen, vorzugsweise von mindestens vier Wochen und besonders bevorzugt von mindestens acht Wochen lagert und wobei die Enzymaktivität vorzugsweise um weniger als 50 %, besonders bevorzugt weniger als 30 % und am meisten bevorzugt um weniger als 20 % bezüglich des Ausgangswerts der Enzymaktivität abnimmt.

Weiterhin kann die Testchemie derart ausgestaltet werden, dass das vorzugsweise mit mindestens einem stabilen Coenzym stabilisierte Enzym bei erhöhten Temperaturen, beispielsweise bei einer Temperatur von mindestens 20 °C, vorzugsweise von mindestens 25 °C und besonders bevorzugt von mindestens 30 °C gelagert wird. Die Enzymaktivität nimmt dabei vorzugsweise um weniger als 50 %, besonders bevorzugt weniger als 30 % und am meisten bevorzugt weniger als 20 % bezüglich ihres Ausgangswerts ab.

Durch die Stabilisierung ist es möglich, das mit einem stabilen Coenzym stabilisierte Enzym auch ohne Trocknungsreagenz für eine lange Zeit, wie oben angegeben, und/oder bei hohen Temperaturen, wie oben angegeben, zu lagern. Weiterhin kann das stabilisierte Enzym auch bei einer hohen relativen Luftfeuchtigkeit, z.B. einer relativen Luftfeuchtigkeit von mindestens 50 % gelagert werden, wobei die Enzymaktivität vorzugsweise um weniger als 50 %, besonders bevorzugt weniger als 30 % und am meisten bevorzugt weniger als 20 % bezüglich des Ausgangswerts abnimmt.

Die Lagerung des mit einem stabilen Coenzym stabilisierten Enzyms kann einerseits als Trockensubstanz und andererseits in Flüssigphase erfolgen. Bevorzugt erfolgt die Lagerung des stabilisierten Enzyms auf oder in einem Testelement, das zur Bestimmung eines Analyten geeignet ist. Das mit einem stabilen Coenzym stabilisierte Enzym ist dabei Bestandteil der bevorzugten Testchemie, welche gegebenenfalls noch weitere Bestandteile wie etwa Salze, Puffer, etc. enthalten kann. Vorzugsweise ist die Testchemie dabei frei von einem Mediator.

Das mit einem stabilen Coenzym stabilisierte Enzym kann allgemein zum Nachweis von Analyten verwendet werden, wobei auf die obige Beschreibung verwiesen werden kann. Beispielsweise können ein oder mehrere Analyte in Körperflüssigkeiten wie etwa Blut, Serum, Plasma oder Urin bzw. in Abwasserproben oder Lebensmitteln nachgewiesen werden.

Als Analyten können beispielsweise beliebige biologische oder chemische Substanzen bestimmt werden, die durch eine Redoxreaktion nachgewiesen werden können, z.B. Substanzen, bei denen es sich um Substrate eines Coenzym-abhängigen Enzyms handelt oder Coenzym-abhängige Enzyme selbst. Bevorzugte Beispiele für Analyten sind Glucose, Milchsäure, Äpfelsäure, Glycerin, Alkohol, Cholesterin, Triglyceride, Ascorbinsäure, Cystein, Glutathion, Peptide, Harnstoff, Ammonium, Salicylat, Pyruvat, 5'-Nukleotidase, Creatinkinase (CK), Lactat-Dehydrogenase (LDH), Kohlendioxid etc. Bevorzugt ist der Analyt Glucose. Besonders bevorzugt ist hierbei der Nachweis von Glucose mit Hilfe von Glucose-Dehydrogenase (GlucDH).

Die Veränderung des stabilen Coenzyms durch Reaktion mit dem Analyten kann grundsätzlich auf beliebige Art und Weise nachgewiesen werden. Hier können grundsätzlich alle aus dem Stand der Technik bekannten Methoden zum Nachweis enzymatischer Reaktionen eingesetzt werden. Vorzugsweise wird jedoch die Veränderung des Coenzyms durch optische Methoden nachgewiesen. Optische Nachweismethoden, die allgemein im Rahmen der Analytmessung eingesetzt werden können, umfassen beispielsweise die Messung einer Remission, einer Absorption, einer Fluoreszenz, eines Circulardichroismus (CD), einer optische Rotationsdispersion (ORD), eine Refraktometrie eine Photometrie oder Kombinationen der genannten und/oder anderer Nachweisverfahren.

Ein optisches Nachweisverfahren, welches im Rahmen der vorliegenden Anmeldung bevorzugt Anwendung findet, ist die Photometrie. Zur photometrischen Messung einer Veränderung des Coenzyms infolge Umsetzung mit dem Analyten bedarf es indessen in der Regel der zusätzlichen Anwesenheit mindestens eines Mediators, welcher die Reaktivität des reduzierten Coenzyms erhöht und eine Übertragung von Elektronen auf einen geeigneten optischen Indikator oder ein optisches Indikatorsystem ermöglicht. Dementsprechend umfasst die Testchemie vorzugsweise weiterhin mindestens einen derartigen Mediator.

Als Mediatoren, welche für die Zwecke der vorliegenden Erfindung geeignet sind, kommen u.a. Nitrosoaniline, wie beispielsweise [(4-Nitrosophenyl)imino]dimethanol-Hydrochlorid, Chinone, wie beispielsweise Phenanthrenchinone, Phenanthrolinchinone oder Benzo[h]-chinolinchinone, Phenazine, wie beispielsweise 1-(3-Carboxypropoxy)-5-ethylphenazinium-trifluormethansulfonat, oder/und Diaphorase (EC 1.6.99.2) in Frage. Bevorzugte Beispiele für Phenanthrolinchinone umfassen 1,10-Phenanthrolin-5,6-chinone, 1,7-Phenanthrolin-5,6-chinone, 4,7-Phenanthrolin-5,6-chinone sowie deren N-alkylierte oder N,N'-dialkylierte Salze, wobei im Falle N-alkylierter bzw. N,N'-dialkylierter Salze Halogenide, Trifluormethansulfonat oder andere die Löslichkeit erhöhende Anionen als Gegenion bevorzugt sind.

Weiterhin kann die Testchemie mindestens einen Indikator enthalten, insbesondere mindestens einen optischen Indikator. Unter einem Indikator wird dabei eine grundsätzlich beliebige Substanz verstanden, welche durch den Ablauf der Nachweisreaktion des Analytnachweises, insbesondere der enzymatischen Reaktion, beeinflusst wird, so dass mindestens eine Eigenschaftsänderung dieser Substanz bei Ablauf der Nachweisreaktion erfasst werden kann. Insbesondere kann diese Eigenschaft eine optische Eigenschaft sein. Dementsprechend kann der Indikator insbesondere mindestens einen Farbstoff umfassen.

Als optischer Indikator oder als optisches Indikatorsystem kann insbesondere eine beliebige Substanz verwendet werden, welche reduzierbar ist und bei Reduktion eine detektierbare Änderung ihrer optischen Eigenschaften, wie beispielsweise Farbe, Fluoreszenz, Remission, Transmission, Polarisation oder/und Brechungsindex, erfährt. Die Bestimmung des Vorhandenseins oder/und der Menge des Analyten in der Probe kann mit dem bloßen Auge oder/und mittels einer Detektionsvorrichtung unter Verwendung eines dem Fachmann geeignet erscheinenden photometrischen Verfahrens erfolgen. Vorzugsweise werden Heteropolysäuren, und insbesondere 2,18-Phosphormolybdänsäure, als optische Indikatoren verwendet, die zum entsprechenden Heteropolyblau reduziert werden.

Besonders bevorzugt wird die Veränderung des Coenzyms durch Messung der Fluoreszenz nachgewiesen. Die Fluoreszenzmessung ist hochsensitiv und ermöglicht den Nachweis selbst geringer Konzentrationen des Analyten in miniaturisierten Systemen.

Alternativ kann die Veränderung des Coenzyms auch elektrochemisch unter Verwendung eines geeigneten Testelements, wie beispielsweise eines elektrochemischen Teststreifens, nachgewiesen werden. Voraussetzung hierfür ist wiederum die Verwendung geeigneter Mediatoren, welche vom reduzierten Coenzym durch Übertragung von Elektronen in eine reduzierte Form überführt werden können. Die Bestimmung des Analyten erfolgt über eine Messung des zur Reoxidation des reduzierten Mediators benötigten Stromes, welcher mit der Konzentration des Analyten in der Probe korreliert. Beispiele für Mediatoren, welche für elektrochemische Messungen verwendet werden können, umfassen insbesondere die vorstehend erwähnten, für photometrische Messungen eingesetzten Mediatoren.

Zum Nachweis eines Analyten kann ein Testelement für einen Flüssigtest verwendet werden, wobei die Testchemie beispielsweise in Form einer Lösung oder Suspension in einer wässrigen oder nichtwässrigen Flüssigkeit oder als Pulver oder Lyophilisat vorliegt. Es kann jedoch auch ein Testelement mit einem Trockentest verwendet werden, wobei das Reagenz beispielsweise auf einem Trägerelement, insbesondere einem Trägerstreifen oder Testträgerband, aufgebracht ist. Der Träger kann beispielsweise einen Teststreifen, umfassend ein saugfähiges oder/und quellbares Material, umfassen, das von der zu untersuchenden Probeflüssigkeit benetzt wird.

Ein besonders bevorzugtes Testformat, umfasst die Verwendung des Enzyms Glucose-Dehydrogenase mit einem stabilen NAD-Derivat zum Nachweis von Glucose, wobei ein Derivat des reduzierten Coenzyms NADH gebildet wird. Der Nachweis von NADH erfolgt durch optische Methoden, z.B. durch photometrische oder fluorometrische Bestimmung nach UV-Anregung. Ein besonders bevorzugtes Testsystem ist in US 2005/0214891 beschrieben, auf das hier ausdrücklich Bezug genommen wird.

Insbesondere kann die Testchemie derart stabil ausgestaltet sein, dass diese ein mit einem stabilen Coenzym stabilisiertes Enzym umfasst, wobei das stabilisierte Enzym bei einer Lagerung von vorzugsweise mindestens zwei Wochen, besonders bevorzugt mindestens vier Wochen und am meisten bevorzugt mindestens acht Wochen bei einer Temperatur von vorzugsweise mindestens 20 °C, besonders bevorzugt mindestens 25 °C und am meisten bevorzugt mindestens 30 °C, gegebenenfalls bei hoher Luftfeuchtigkeit und ohne Trockenreagenz eine Abnahme der enzymatischen Aktivität von weniger als 50 %, vorzugsweise weniger als 30 % und am meisten bevorzugt weniger als 20 % gegenüber dem Ausgangswert aufweist.

Auch andere Arten stabiler Testchemien können alternativ oder zusätzlich verwendet werden, beispielsweise die in WO 2007/012494 A1 beschriebene Testchemie. Die Testchemie kann grundsätzlich in beliebiger Weise in einem Testelement enthalten sein. Die Testchemie bzw. das Testelement können zur Durchführung von Trocken- oder Flüssigtests geeignet sein. Beispielsweise kann die Testchemie zu diesem Zweck auf einem geeigneten Trägermaterial aufgebracht sein, beispielsweise auf einem Kunststoff und/oder einem keramischen Material und/oder einem Papiermaterial.

Wie oben ausgeführt, kann die Qualität der Testchemie, auf welche bei der Qualitätsmessung geschlossen wird, insbesondere mindestens eine Information über eine Aktivität des optional in der Testchemie enthaltenen mindestens einen Enzyms und/oder eines Coenzyms umfassen. Die Qualität kann unmittelbar die Aktivität des Enzyms oder Coenzyms sein oder kann eine andere Information sein oder umfassen, welche aus der Aktivität des Enzyms oder Coenzyms abgeleitet ist oder aus welcher sich auf die Aktivität des Enzyms oder Coenzyms schließen lässt.

In einem weiteren Aspekt der vorliegenden Erfindung wird ein Verfahren zum Nachweis mindestens eines Analyten in einer Probe vorgeschlagen. Das Verfahren kann insbesondere unter Verwendung eines erfindungsgemäßen Analysegeräts in einer oder mehreren der dargestellten Ausgestaltungen durchgeführt werden, so dass bezüglich möglicher optionaler Ausgestaltungen und/oder bezüglich möglicher Definitionen der verwendeten Begriffe des Verfahrens auf die Beschreibung des Analysegeräts verwiesen werden kann.

Das Verfahren umfasst die folgenden Verfahrensschritte, welche möglicherweise, jedoch nicht notwendigerweise, in der dargestellten Reihenfolge durchgeführt werden können. Weiterhin können auch einzelne, mehrere oder alle der Verfahrensschritte wiederholt durchgeführt werden, und es können mehrere Verfahrensschritte zeitlich überlappend oder zeitgleich durchgeführt werden.

So wird mindestens eine Analytmessung durchgeführt, wobei bezüglich der möglichen Ausgestaltungen dieser Analytmessung weitgehend auf die obige Beschreibung verwiesen werden kann. Insbesondere kann bei der Analytmessung mindestens eine durch Anwesenheit des Analyten veränderliche Eigenschaft mindestens einer Testchemie eines Testelements erfasst werden, insbesondere eine elektrische und/oder optische Eigenschaft. Diese Analytmessung kann grundsätzlich durch ein Analysegerät gemäß der obigen Beschreibung durchgeführt werden, beispielsweise durch mindestens einen Analytdetektor und insbesondere mindestens einen optischen Analytdetektor eines derartigen Analysegeräts. Alternativ oder zusätzlich kann die Analytmessung jedoch auch grundsätzlich durch einen Benutzer ohne Verwendung eines Analytdetektors durchgeführt werden. So kann beispielsweise ein Testelement verwendet werden, beispielsweise ein Teststreifen, Teststäbchen oder Testband, welches mindestens eine Testchemie aufweist, wobei ein Benutzer beispielsweise eine Verfärbung der Testchemie, beispielsweise mindestens eines Testfelds, mit dem Auge detektiert. Beispielsweise kann der Benutzer dabei einen Vergleich mit einer vorgegebenen Farbskala durchführen. Derartige Tests sind grundsätzlich auch kommerziell erhältlich.

Weiterhin umfasst das Verfahren mindestens einen Verfahrensschritt einer Qualitätsmessung an der Testchemie. Bezüglich möglicher Ausgestaltungen dieser Qualitätsmessung kann insbesondere auf die obige Beschreibung verwiesen werden. Bei der Qualitätsmessung wird mindestens eine Eigenlumineszenz der Testchemie erfasst, beispielsweise mindestens eine Eigenfluoreszenz. Aus der Eigenlumineszenz wird auf eine Qualität der Testchemie geschlossen, insbesondere auf eine Degradation der Testchemie. Die Qualitätsmessung kann vorzugsweise unter Verwendung mindestens eines Qualitätsdetektors durchgeführt werden, welcher beispielsweise gemäß der obigen Beschreibung möglicher Qualitätsdetektoren ausgestaltet sein kann, jedoch beispielsweise auch unabhängig von dem optionalen Analytdetektor realisierbar ist. So kann beispielsweise ein Qualitätsdetektor mit den oben genannten, einen Qualitätsdetektor betreffenden Merkmalen als separates Gerät vorgesehen sein. Der Qualitätsdetektor kann beispielsweise als Handgerät geliefert werden, beispielsweise mit einem Volumen von nicht mehr als 100 cm³, vorzugsweise von nicht mehr als 50 cm³, so dass dieser beispielsweise als Taschengerät ausgestaltet werden kann, um die Qualität von Testelementen, beispielsweise Teststreifen, zu überprüfen. Bezüglich möglicher Ausgestaltungen des Qualitätsdetektors, insbesondere bezüglich möglicher Komponenten desselben, kann auf die obige Beschreibung verwiesen werden. Der Qualitätsdetektor kann insbesondere eine eigene Auswertevorrichtung umfassen, beispielsweise mindestens eine Datenverarbeitungsvorrichtung und/oder mindestens einen flüchtigen und/oder nicht-flüchtigen Datenspeicher. Der Qualitätsdetektor kann insbesondere mindestens eine Anzeigevorrichtung aufweisen, welche eingerichtet ist, um einem Benutzer mindestens ein Ergebnis der Qualitätsmessung mitzuteilen. Diese Anzeigevorrichtung kann beispielsweise optischer, akustischer, oder haptischer Natur sein, so dass entsprechend Informationen über das Ergebnis der Qualitätsmessung an den Benutzer übermittelt werden können. So kann beispielsweise auch bei Einzelteststreifen, welche für eine Ablesung entsprechend einer Farbskala, ohne Verwendung eines Analytdetektors, eingerichtet sind, vor Verwendung der Teststreifen eine Qualitätsmessung der beschriebenen Art durchgeführt werden. Auf diese Weise kann auch bei einzelnen Testelementen eine Verwendung degradierter Testelemente für einen Analytnachweis verhindert oder zumindest vermieden werden. Ein derartiger Qualitätsdetektor wird somit in einem weiteren Aspekt der vorliegenden Erfindung als unabhängiger Gegenstand vorgeschlagen.

In einem weiteren Aspekt der vorliegenden Erfindung wird eine Verwendung eines Qualitätsdetektors und/oder eines Analysegeräts gemäß der vorliegenden Erfindung zur Vermeidung von Analytmessungen unter Verwendung von Testelementen mit degradierter Testchemie vorgeschlagen.

In einem weiteren Aspekt der vorliegenden Erfindung wird eine Verwendung einer Messung einer Eigenlumineszenz einer Testchemie eines Testelements zur Erkennung degradierter Testelemente vorgeschlagen.

Die erfindungsgemäß vorgeschlagenen Vorrichtungen, Verfahren und Verwendungen weisen gegenüber bekannten Vorrichtungen, Verfahren und Verwendungen eine Vielzahl von Vorteilen auf. So ermöglicht die vorliegende Erfindung insbesondere, dass zuverlässig und sicher, beispielsweise auch durch einen Benutzer, erkannt werden kann, ob eine Testelement - ggf. trotz hoher Langzeitstabilität - nicht doch verfallen ist oder in nicht tragbarem Maße degradiert sein könnte. Experimentelle Untersuchungen, welche nachfolgend exemplarisch noch näher vorgestellt werden, haben dabei gezeigt, dass in vielen Fällen in der Testchemie das Element mit geringster Stabilität das Enzym ist, welches degradieren kann, wobei eine Enzymaktivität absinken kann. Die vorliegende Erfindung stellt insbesondere Verfahren bereit, mittels derer eine Degradation des Enzyms direkt gemessen werden kann.

Im Vergleich zu den in EP 1 189 064 A1 und in EP 2 221 608 A1 beschriebenen Verfahren sind das erfindungsgemäß vorgeschlagene Analysegerät und das erfindungsgemäß vorgeschlagene Verfahren nicht auf die Erfassung eines Trockenleerwerts angewiesen. Eine Trockenleerwertmessung einer Remission zum Ausschluss grob degradierter Testelemente kann jedoch zusätzlich optional vorgesehen sein. Das vorgeschlagene Verfahren, bei welchem die Eigenlumineszenz der Testchemie, insbesondere einer Eigenfluoreszenz mindestens eines optional in der Testchemie enthaltenen Enzyms und/oder Coenzyms erfasst wird, ermöglicht hingegen eine erheblich präzisere Erfassung von Degradationsvorgängen, welche unmittelbar die an dem Analytnachweis beteiligte Komponente oder die an dem Analytnachweis beteiligten Komponenten betreffen können. Durch die Erfassung der Eigenlumineszenz, welche vorzugsweise in mindestens zwei verschiedenen Wellenlängenbereichen erfolgen kann, beispielsweise in Form einer ersten Eigenfluoreszenz integral in einem ersten Wellenlängenbereich und einer zweiten Eigenfluoreszenz integral in einem zweiten Wellenlängenbereich, kann eine interne Referenzierung des Verfahrens realisiert werden. Auf diese Weise lässt sich, beispielsweise durch Quotientenbildung oder durch andere Auswertungsverfahren, eine Referenzierung auf einen aufwendig beizufügenden Chargenkontrollwert vermeiden, obgleich eine derartige Referenzierung optional zusätzlich durchführbar ist. Auf diese Weise lässt sich beispielsweise vermeiden, dass einer Charge von Testelementen, beispielsweise einer Charge von Teststreifen oder einer Bandkassette, ein Datenspeicher beigefügt werden muss, welcher den Chargenkontrollwert enthält. Insgesamt lässt sich somit das Verfahren, welches erfindungsgemäß ausgestaltet ist, im Vergleich zum Stand der Technik erheblich sicherer und einfacher gestalten.

Bevorzugt wird bei der Qualitätsmessung eine Eigenlumineszenz des Enzyms direkt gemessen. Alternativ könnte der Testchemie jedoch auch eine Substanz beigemischt werden, die, wie das Enzym, unter gleicher Temperaturbelastung und/oder Feuchtebelastung ähnlich degradiert wie das Enzym, und diese Degradation könnte gesondert nachgewiesen werden.

Klassisch werden zur Messung einer Degradation von Enzymen Aktivitätsbestimmungen herangezogen, beispielsweise gemäß der obigen Beschreibung. Bei dieser können beispielsweise Eluate des Testelements, beispielsweise des Teststreifens, erzeugt werden, und eine Aktivität der hierin enthaltenen Enzyme kann, beispielsweise über eine Zugabe eines Coenzyms und eines Analyten, bestimmt werden. Hierbei handelt es sich jedoch um ein laboranalytisches Verfahren, welches in der Regel nicht mit einfachen Mitteln von einem Leihen durchgeführt werden kann. Weiterhin ist ein derartiges Verfahren in der Regel aufwendig und erfordert eine Zerstörung des Testelements bei der Qualitätsmessung, so dass der ursprüngliche Verwendungszweck, d.h. der Analytnachweis und insbesondere eine Glucosebestimmung, nicht mehr möglich ist. Besonders bevorzugt ist es daher, wenn die Qualitätsmessung und der Qualitätsdetektor derart ausgestaltet sind, dass die Qualitätsmessung zerstörungsfrei erfolgt. Dies lässt sich bei der vorgeschlagenen Messung der Eigenlumineszenz besonders einfach realisieren.

Erfindungsgemäß wird somit eine einfache, praktikable und nicht-destruktive Methode zum Nachweis einer Alterung des Testelements bereitgestellt, ggf. insbesondere einer Enzymdegradation. Die Qualitätsmessung kann insbesondere direkt erfolgen, da über die Lumineszenzmessung unmittelbar ein Nachweis einer Enzymdegradation erfolgen kann, im Gegensatz beispielsweise zu indirekten Methoden. Weiterhin erfordert die vorgeschlagene Lumineszenzmessung in der Regel keine Modifikation einer Testchemierezeptur.

Erfindungsgemäß wurde, wie oben ausgerührt und wie nachfolgend exemplarisch noch näher beschrieben, eine zunächst überraschende Eigenschaft üblicher Testchemien festgestellt, nämlich dass mit einer Degradation, insbesondere bei enzymatischen Nachweisreaktionen, eine Veränderung der intrinsischen Lumineszenz und insbesondere der intrinsischen Fluoreszenz der Testchemie vor der Benetzung einhergeht. Insbesondere kann es sich hierbei um eine erhöhte Autofluoreszenz von Glucose-Dehydrogenase handeln. Diese veränderte intrinsische Lumineszenz oder Eigenlumineszenz kann erfindungsgemäß zur Erkennung einer Testelementdegradation oder allgemein zur Qualitätsbestimmung der Testchemie oder des gesamten Testelements herangezogen werden.

Obgleich im Rahmen der vorliegenden Erfindung ein Schwerpunkt in einer Degradationserkennung an Glucosemessstreifen im Rahmen der oben beschriebenen cNAD-Testchemie liegt, sind das vorgeschlagene Verfahren und das vorgeschlagene Analysegerät grundsätzlich auf eine Vielzahl von Testelementen und ggf. auch auf eine Verfallserkennung ganz allgemein von Testsystemen, z.B. Reagenzien-Kits, erweiterbar.

Die Verwendung beispielsweise von Fluorophoren zur Erkennung von Glucosekonzentrationen in Teststreifen ist allgemein aus dem Stand der Technik bekannt, beispielsweise aus EP 1 780 288 B1, aus WO 2009/015870 A1 oder aus weiteren der oben genannten Dokumente des Standes der Technik. Glucose-induzierte Veränderungen in der Fluoreszenz von Proteinen und anderen Fluorophoren werden beispielsweise auch in J. C. Pickup et al. Biosensors and Bioelectronics 20 (2005), 2555, beschrieben. Dementsprechend ist im Rahmen der vorliegenden Erfindung als überraschend zu bezeichnen, dass allgemein festgestellt wurde, dass Lumineszenzänderungen, insbesondere Fluoreszenzänderungen, beobachtbar sind, die gerade nicht auf den Nachweis des Analyten, sondern beispielsweise auf eine Degradation und insbesondere einen Abfall beispielsweise einer Enzymaktivität zurückzuführen sind und mit diesem Abfall korrelieren. Auch in C. M. Moore, Biomacromolecules 5, (2004), 1241, wird beispielsweise auf Seite 1243 unter der Überschrift "Enzyme Lifetime Studies" beschrieben, dass die Lebensdauer von Alkohol-Dehydrogenase bezüglich ihrer Aktivität bestimmt wird, in diesem Fall durch Zugabe von NAD+, wobei hier jedoch die Fluoreszenz des erst bei der eigentlichen Analytnachweisreaktion entstehenden Coenzyms NADH gemessen wird, nicht jedoch diejenige des Proteins selbst. Insofern ist bereits eine Benetzung der Testchemie mit der Probe erforderlich, um die Qualitätsmessung durchführen zu können, im Gegensatz zu der erfindungsgemäßen Ausgestaltung, bei welcher die Eigenlumineszenz der Testchemie erfasst wird. Somit ist im Rahmen der vorliegenden Erfindung insbesondere eine rechtzeitige Erkennung einer Degradation möglich, bevor das Testelement mit der Probe in Kontakt gebracht wird, so dass sich beispielsweise eine wiederholte Probengenerierung, beispielsweise durch Perforation einer Hautpartie, erfindungsgemäß bei Erkennung degradierter Testelemente rechtzeitig vermeiden lässt. Hierdurch ergibt sich ein erheblicher Gewinn an Komfort für den Benutzer der Testelemente.

Insgesamt lassen sich mit der vorgeschlagenen Erfindung Analysegeräte und Verfahren zum Analytnachweis realisieren, welche sicher und dennoch einfach ausgestaltet werden können und welche zuverlässig eine Verwendung degradierter Testelemente verhindern können. Auf diese Weise kann eine Betriebssicherheit deutlich erhöht werden, und das Risiko einer fehlerhaften Diagnose aufgrund einer Verwendung degradierter Teststreifen lässt sich deutlich vermindern.

Zusammenfassend werden im Rahmen der vorliegenden Erfindung folgende Ausführungsformen als besonders bevorzugt erachtet:
Ausführungsform 1: Analysegerät zum Nachweis mindestens eines Analyten in einer Probe, insbesondere zum Nachweis von Blutglukose,
   - wobei das Analysegerät mindestens ein Testelement aufweist, wobei das Testelement mindestens eine Testchemie aufweist, wobei das Testelement mindestens ein Testfeld aufweist, wobei das Testfeld ein Bereich ist, in dem mindestens eine zusammenhängende Schicht der Testchemie auf ein Trägerelement aufgebracht oder in ein Trägerelement eingebracht ist, wobei das Analysegerät eingerichtet ist, um mittels des Testelements mindestens eine Analytmessung durchzuführen, wobei die Testchemie eine Substanz oder ein Substanzgemisch ist, welches eingerichtet ist, um bei Anwesenheit des Analyten mindestens eine nachweisbare veränderliche Eigenschaft zu verändern, insbesondere eine elektrische und/oder optische Eigenschaft, wobei bei der Analytmessung die mindestens eine durch Anwesenheit des Analyten veränderliche Eigenschaft der mindestens einen Testchemie des Testelements erfasst wird, und
   - wobei das Analysegerät weiterhin eingerichtet ist, um mindestens eine Qualitätsmessung an der Testchemie durchzuführen, wobei bei der Qualitätsmessung mindestens eine Eigenlumineszenz der Testchemie erfasst wird und aus der Eigenlumineszenz auf eine Qualität der Testchemie geschlossen wird, wobei die Qualität der Testchemie eine Information ist, welche Aufschluss über einen Zustand der Testchemie gibt, wobei die Qualität mindestens eine Qualitätsinformation umfasst, welche eine Degradation oder einen Alterungszustand der Testchemie quantifiziert,
   wobei das Analysegerät eingerichtet ist, um die Qualitätsmessung mindestens einmal vor der Analytmessung durchzuführen, wobei das Analysegerät eingerichtet ist, um entsprechend der erfassten Qualität mindestens eine Aktion durchzuführen, ausgewählt aus der Gruppe bestehend aus: einer Ausgabe eines Hinweises an einen Benutzer, insbesondere eines Wamhinweises; einer Weitergabe mindestens einer Information über die Qualität an mindestens ein weiteres Gerät; einer Abspeicherung mindestens einer Information über die Qualität in einem Datenspeicher; einer Verhinderung oder Ermöglichung der Analytmessung; einer Berücksichtigung oder Nichtberücksichtigung einer bereits erfolgten Analytmessung; einer Berechnung einer Konzentration des Analyten in der Probe aus der erfassten Eigenschaft unter Berücksichtigung der Qualität der Testchemie, insbesondere unter Verwendung einer die Qualität berücksichtigenden Korrektur.
Ausführungsform 2: Analysegerät der vorhergehenden Ausführungsform, wobei das Analysegerät mindestens einen optischen Analytdetektor umfasst und eingerichtet ist, um bei der Analytmessung mittels des optischen Analytendetektors eine optische Erfassung der Eigenschaft durchzuführen, insbesondere eine Farbmessung und/oder eine Remissionsmessung und/oder eine Fluoreszenzmessung.
Ausführungsform 3: Analysegerät nach einer der vorhergehenden Ausführungsformen, wobei die Eigenlumineszenz mindestens eine Eigenfluoreszenz der Testchemie umfasst.
Ausführungsform 4: Analysegerät nach einer der vorhergehenden Ausführungsformen, wobei das Analysegerät eingerichtet ist, um auf eine Degradation des Testelements zu schließen, wenn die Eigenlumineszenz mindestens eine vorgegebene Schwelle überschreitet.
Ausführungsform 5: Analysegerät nach einer der vorhergehenden Ausführungsformen, wobei das Analysegerät weiterhin mindestens einen optischen Qualitätsdetektor umfasst und eingerichtet ist, um bei der Qualitätsmessung mittels des optischen Qualitätsdetektors die Messung der Eigenlumineszenz der Testchemie durchzuführen.
Ausführungsform 6: Analysegerät nach der vorhergehenden Ausführungsform, wobei das Analysegerät eingerichtet ist, um mittels des optischen Qualitätsdetektors die Eigenlumineszenz in mindestens zwei verschiedenen Wellenlängenbereichen zu erfassen, insbesondere mindestens eine erste Eigenlumineszenz oder mindestens ein erstes Eigenlumineszenzspektrum in einem ersten Wellenlängenintervall und mindestens eine zweite Eigenlumineszenz oder mindestens ein zweites Eigenlumineszenzspektrum in mindestens einem zweiten Wellenlängenintervall.
Ausführungsform 7: Analysegerät nach der vorhergehenden Ausführungsform, wobei das Analysegerät eingerichtet ist, um aus der Eigenlumineszenz in den mindestens zwei verschiedenen Wellenlängenbereichen mindestens eine die Qualität charakterisierende Qualitätskennzahl zu berechnen, insbesondere durch Quotientenbildung und/oder Bildung einer Linearkombination.
Ausführungsform 8: Analysegerät nach einer der beiden vorhergehenden Ausführungsformen, wobei das Analysegerät eingerichtet ist, um eine erste Eigenlumineszenz integral in einem ersten Wellenlängenbereich von 380 nm bis 420 nm zu erfassen und um eine zweite Eigenlumineszenz integral in einem zweiten Wellenlängenbereich von mindestens 420 nm oder über 420 nm, beispielsweise in einem zweiten Wellenlängenbereich von 420 nm bis 650 nm, zu erfassen.
Ausführungsform 9: Analysegerät nach einer der drei vorhergehenden Ausführungsformen, wobei der Qualitätsdetektor mindestens eine Anregungslichtquelle aufweist, wobei die Anregungslichtquelle eingerichtet ist, um die Testchemie mit einem Anregungslicht mit einer Anregungswellenlänge von 340 nm bis 380 nm zu bestrahlen.
Ausführungsform 10: Analysegerät nach einer der vorhergehenden Ausführungsformen, weiterhin umfassend eine Auswertevorrichtung, wobei die Auswertevorrichtung eingerichtet ist, um die Qualität mit mindestens einer Bedingung zu vergleichen, insbesondere um die Qualität mit mindestens einem Schwellwert zu vergleichen.
Ausführungsform 11: Analysegerät nach einer der vorhergehenden Ausführungsformen, wobei die Testchemie mindestens ein Enzym umfasst.
Ausführungsform 12: Analysegerät nach der vorhergehenden Ausführungsform, wobei das Enzym ausgewählt ist aus der Gruppe bestehend aus einer Oxidase und einer Dehydrogenase.
Ausführungsform 13: Analysegerät nach einer der beiden vorhergehenden Ausführungsformen, wobei das Enzym ausgewählt ist aus der Gruppe bestehend aus: Glucose-Dehydrogenase (E.C.1.1.1.47); Lactat-Dehydrogenase (E.C.1.1.1.27, 1.1.1.28); Malat-Dehydrogenase (E.C.1.1.1.37); Glycerin-Dehydrogenase (E.C.1.1.1.6); Alkohol-Dehydrogenase (E.C.1.1.1.1); alpha-Hydroxybutyrat-Dehydrogenase; Sorbitol-Dehydrogenase; Aminosäure-Dehydrogenase, insbesondere L-Aminosäure-Dehydrogenase (E.C.1.4.1.5); Glucoseoxidase (E.C.1.1.3.4); Cholesterinoxidase (E.C.1.1.3.6); Aminotransferasen, insbesondere Aspartat oder Alanin Aminotransferase; 5'-Nukleotidase; Creatin-Kinase; Glucose 6-Phosphat-Dehydrogenase (EC 1.1.1.49); NAD-abhängiger Cholesterol-Dehydrogenase (EC 1.1.1.62); FAD-abhängiger Glucose-Dehydrogenase (EC 1.1.99.10); PQQ-abhängiger Glucose-Dehydrogenase (EC1.1.5.2).
Ausführungsform 14: Analysegerät nach einer der drei vorhergehenden Ausführungsformen, wobei die Qualität mindestens eine Information über eine Aktivität des Enzyms umfasst.
Ausführungsform 15: Verfahren zum Nachweis mindestens eines Analyten in einer Probe, insbesondere unter Verwendung eines Analysegeräts nach einer der vorhergehenden Ausführungsformen wobei das Verfahren folgende Schritte umfasst:
   - mindestens eine Analytmessung, wobei bei der Analytmessung mindestens eine durch Anwesenheit des Analyten veränderliche Eigenschaft mindestens einer Testchemie eines Testelements erfasst wird, insbesondere eine elektrische und/oder optische Eigenschaft, wobei das Testelement mindesten eine Testchemie aufweist, wobei das Testelement mindestens ein Testfeld aufweist, wobei das Testfeld ein Bereich ist, in dem mindestens eine zusammenhängende Schicht der Testchemie auf ein Trägerelement aufgebracht oder in ein Trägerelement eingebracht ist, und
   - mindestens eine Qualitätsmessung an der Testchemie, wobei bei der Qualitätsmessung mindestens eine Eigenlumineszenz der Testchemie erfasst wird und aus der Eigenlumineszenz auf eine Qualität der Testchemie geschlossen wird, wobei die Qualität mindestens eine Qualitätsinformation umfasst, welche eine Degradation oder einen Alterungszustand der Testchemie quantifiziert,
   wobei entsprechend der erfassten Qualität mindestens eine Aktion durchgeführt wird, ausgewählt aus der Gruppe bestehend aus: einer Ausgabe eines Hinweises an einen Benutzer, insbesondere eines Warnhinweises; einer Weitergabe mindestens einer Information über die Qualität an mindestens ein weiteres Gerät; einer Abspeicherung mindestens einer Information über die Qualität in einem Datenspeicher; einer Verhinderung oder Ermöglichung der Analytmessung; einer Berücksichtigung oder Nichtberücksichtigung einer bereits erfolgten Analytmessung; einer Berechnung einer Konzentration des Analyten in der Probe aus der erfassten Eigenschaft unter Berücksichtigung der Qualität der Testchemie, insbesondere unter Verwendung einer die Qualität berücksichtigenden Korrektur.
Ausführungsform 16: Verwendung eines Analysegeräts nach einer der vorhergehenden, ein Analysegerät betreffenden Ausführungsformen zur Vermeidung von Analytmessungen unter Verwendung von Testelementen mit degradierter Testchemie.

### Kurze Beschreibung der Figuren

Weitere Einzelheiten und Merkmale der Erfindung ergeben sich aus der nachfolgenden Beschreibung von bevorzugten Ausführungsbeispielen, insbesondere in Verbindung mit den Unteransprüchen. Hierbei können die jeweiligen Merkmale für sich alleine oder zu mehreren in Kombination miteinander verwirklicht sein. Die Erfindung ist nicht auf die Ausführungsbeispiele beschränkt. Die Ausführungsbeispiele sind in den Figuren schematisch dargestellt. Gleiche Bezugsziffern in den einzelnen Figuren bezeichnen dabei gleiche oder funktionsgleiche beziehungsweise hinsichtlich ihrer Funktionen einander entsprechende Elemente.

Im Einzelnen zeigen:
- Figur 1: Ein erstes Ausführungsbeispiel eines erfindungsgemäßen Analysegerät;
- Figur 2: Ein Ausführungsbeispiel eines erfindungsgemäßen Verfahrens;
- Figur 3: Eine Eigenfluoreszenz eines Testelements nach verschiedenen Alterungsprozessen;
- Figuren 4A bis 4H: Eigenfluoreszenzen von Testelementen nach verschiedenen Lagerungen bei verschiedenen Anregungswellenlängen;
- Figur 5: Ein Zusammenhang zwischen einer Enzymaktivität und einem Verhältnis einer integralen Intensität der Eigenfluoreszenz bei Wellenlängen kleiner 420 nm zur integralen Intensität der Eigenfluoreszenz bei Wellenlängen größer 420 nm bei einer Anregungswellenlänge von 360 nm.
- Figuren 6A bis 6C: Fluoreszenzspektren verschiedener vollständig oder teilweise aufgebauter Testelemente vor und nach einer Alterung;
- Figur 7: Ein zu Figur 1 alternative Ausführungsbeispiel eines Analysegeräts; und
- Figur 8: Eine schematische Darstellung eines Schichtaufbaus eines Testelements.

### Ausführungsbeispiele

### Testelemente:

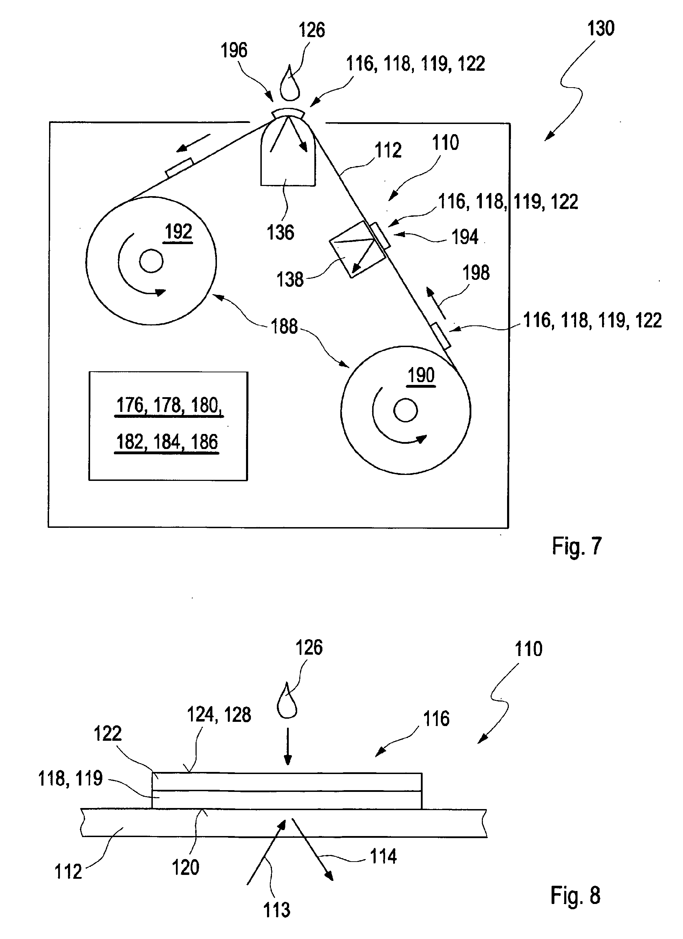

In Figur 8 ist schematisch ein möglicher Aufbau eines Testelements 110 in einer Schnittdarstellung gezeigt, welcher auch im Rahmen der vorliegenden Erfindung zum Einsatz kommen kann. Das Testelement 110 umfasst ein Trägerelement 112, welches beispielsweise als Streifen ausgestaltet sein kann. Beispielsweise kann als Trägerelement, wie im Folgenden exemplarisch angenommen, eine Kunststofffolie verwendet werden, beispielsweise ein Polycarbonat, beispielsweise Pokalon®. Insgesamt kann das Testelement 110 beispielsweise als Teststreifen oder Testband ausgestaltet werden. Auch andere Ausgestaltungen sind jedoch möglich. Das Trägerelement 112 kann beispielsweise ganz oder teilweise transparent ausgestaltet sein, so dass in Figur 8 eingestrahltes Licht 113 und detektierbares Licht 114 das Trägerelement durchdringen können.

Auf das Trägerelement 112 ist ein Schichtaufbau aufgebracht. Dieser umfasst in dem dargestellten Ausführungsbeispiel optional zwei Schichten und bildet ein Testfeld 116. Dieses Testfeld 116 umfasst in dem dargestellten Ausführungsbeispiel exemplarisch eine Nachweisschicht 118, welches eine Testchemie 119 umfasst, mit einer dem Trägerelement 112 zuweisenden Nachweisseite 120. Weiterhin umfasst das Testfeld 116 optional in dem dargestellten Ausführungsbeispiel eine Abtrennschicht 122 auf der dem Trägerelement 112 abgewandten Seite der Nachweisschicht 118. Diese Abtrennschicht 122 dient zum Abtrennen störender Bestandteile einer Probe 126 einer Körperflüssigkeit, welche auf einer Aufgabenseite 128 auf eine Testfeldoberfläche 124 aufgebracht werden kann, beispielsweise zum Abtrennen von Erythrozyten.

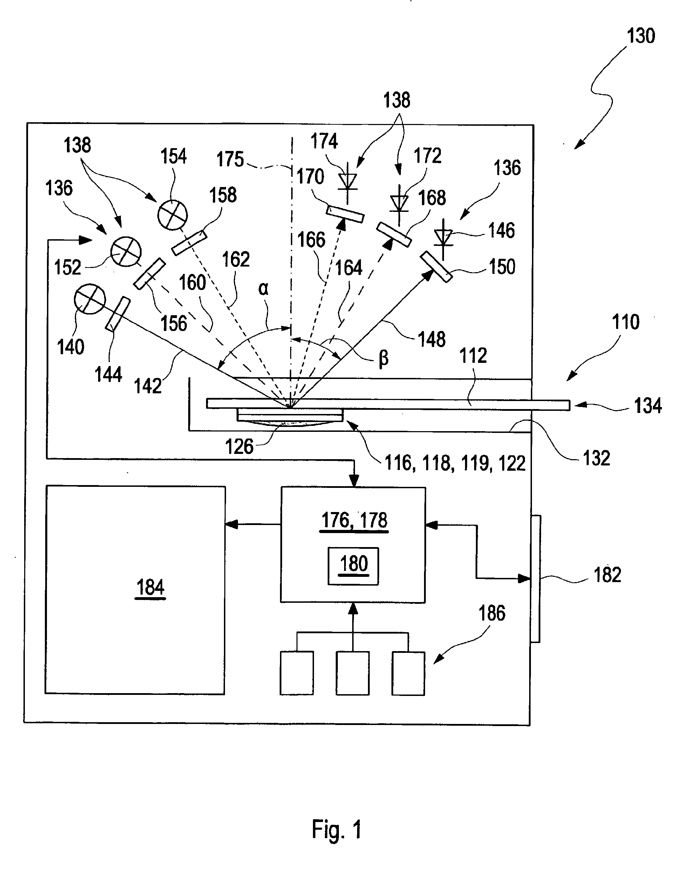

Es wird darauf hingewiesen, dass der in Figur 8 dargestellte Aufbau des diagnostischen Testelements lediglich exemplarisch zu verstehen ist und dass auch andere Arten von Aufbauten möglich sind. So können beispielsweise mehrere Nachweisschichten 118 und/oder mehrere Abtrennschichten 122 oder gar keine Abtrennschicht 122 vorgesehen sein. Weiterhin kann der in Figur 1 gezeigte Aufbau durch verschiedene weitere Elemente, die nicht dargestellt sind, ergänzt werden. So kann beispielsweise auf der Testfeldoberfläche 124 ein Spreitnetz vorgesehen sein. Weiterhin können Teile der Testfeldoberfläche 124 abgedeckt sein, beispielsweise mit einem hydrophoben Material, beispielsweise um lediglich einen Teil der Aufgabenseite 128 für eine Beaufschlagung mit der Probe 126 zugänglich zu gestalten. Für mögliche Ausgestaltungen des diagnostischen Testelements 110 kann beispielsweise auf die oben genannte EP 0 821 234 B1 oder auf andere bekannten Testreifenaufbauten verwiesen werden.

### Herstellungsbeispiel

Im vorliegenden Beispiel werden Schichtaufbauten des Testfelds 116 verwendet, die wie folgt hergestellt sind:

### a) Nachweisschicht:

Zur Herstellung einer Dispersion für die Nachweisschicht 118 werden zunächst zwei Teillösungen (Teillösung 1 und 2) hergestellt, diese dann zu einem Teilansatz vereinigt. Der Begriff "Lösung" wird in diesem Zusammenhang unabhängig davon verwendet, ob tatsächlich eine echte Lösung vorliegt oder lediglich beispielsweise eine Dispersion. Eine Enzymlösung wird hergestellt, und der Teilansatz 1 und die Enzymlösung werden gemischt, so dass eine Beschichtungsmasse entsteht. Hierzu wird wie folgt vorgegangen:
Teillösung 1: 0,34 g Xanthangum werden in 35,5 g 0,02 M Glycerin-3-phosphat-Puffer von pH 6,5 24 h vorgequollen und mit 5,0 g Polyvinylpropionat-Dispersion gemischt.
Teillösung 2: 5,2 g Transpafill werden in 21,5 g Wasser 10 min mit einem Ultraturrax dispergiert.
Teilansatz 1: Beide Teillösungen werden vereinigt und nach Zugabe von 0,15 g Tetraethylammoniumchlorid, 0,17 g N-Octanoyl-N-methyl-glucamid, 0,06 g N-Methyl-N-octadecenyl-taurat ("Geropon T 77") und 0,88 g PVP (MG 25.000) 1 h mit einem Flügelrührer mäßig gerührt. Dann werden der Reihe nach folgende Teillösungen hinzugegeben:
   - 0,10 g Bis-(2-hydroxyethyl)-(4-hydrox-iminocyclohexa-2,5-dienylidin)-ammoniumchlorid in 1,5 g Wasser,
   - 0,65 g 2,18-Phosphormolybdänsäure-hexanatriumsalz in 1,5 g Wasser,
   worauf der pH mit NaOH auf 6,7 eingestellt wird.
Enzymlösung: Zu 25,6 g 0,1 M Glycerin-3-phosphat-Puffer von pH 6,5 werden 5 mg PQQ-di-Natriumsalz und 0,28 g GDH (Mutante 31) sowie 0,16 g einer 1 M CaCl₂-Lösung gegeben und > 3 h gerührt.
Teilansatz 1 und Enzymlösung werden gemischt, mit einer Lösung von 20 mg K₃[Fe(CN)₆] in 0,4 g Wasser sowie 1,0 g 2-Methyl-2-butanol versetzt und 30 min gerührt. Hierbei entsteht eine Beschichtungsmasse zur Herstellung der Nachweisschicht 118.

Die derart hergestellte Beschichtungsmasse wird mit einem Flächengewicht von 90 g/m² auf eine Trägerfolie 119 in Form einer Polycarbonatfolie mit einer Dicke von 125 Mikrometern aufgetragen und getrocknet.

Bei Transpafill^{®} handelt es sich um ein kommerziell erhältliches Natriumaluminiumsilikatpulver der Evonik Industries AG. Die präzisionsverbessernde Wirkung von N-Methyl-N-octadecenyl-taurat ("Geropon T 77") wurde in EP 0 995 994 beschrieben.

### b) Abtrennschicht:

Auch zur Herstellung der Abtrennschicht 122 werden in dem vorliegenden Ausführungsbeispiel zunächst zwei Teillösungen (Teillösung 1 und Teillösung 2) hergestellt und diese dann vereinigt. Hierbei wird wie folgt vorgegangen:
Teillösung 1: Eine Aufschlämmung von 1,37 g Gantrez S 97 in 13,5 g Wasser wird mit 2,2 g 16 %-iger NaOH versetzt und über Nacht vorgequollen. Dann werden 0,40 g Tetraethylammoniumchlorid, 0,34 g N-Octanoyl-N-methyl-glucamid, 0,06 g N-Methyl-N-octadecenyl-taurat ("Geropon T 77") und 1,87 g PVP (MG 25.000) hinzugegeben und 1 h gerührt.
Teil"lösung" 2: 14,3 g Titandioxid E 1171 der Fa. Kronos und 1,95 g Fällungskieselsäure FK 320 der Fa. Degussa werden in 36,4 g Wasser 10 min mit einem Ultraturrax dispergiert.

Nach Vereinigen der Teillösungen werden 5,7 g Polyvinylpropionat-Dispersion, 0,15 g Bis-(2-hydroxyethyl)-(4-hydrox-iminocyclohexa-2,5-dienylidin)-ammoniumchlorid in 4,2 g Wasser, 1,85 g 2,18-Phosphormolybdänsäure-hexanatriumsalz in 4,2 g Wasser, 10 mg K₃[Fe(CN)₆] in 0,4 g Wasser hinzugefügt und mit NaOH auf pH 6,8 gestellt. Nach Zugabe von 1,0 g 2-Methyl-2-butanol noch 1 h gerührt.

Bei der Bezeichnung Gantrez^{®} handelt es sich um eine Produktbezeichnung der ISP International Speciality Products, Köln, Deutschland. Chemisch handelt es sich dabei um ein Copolymer von Maleinsäure und Methylvinylether.

Die derart durch Vereinigung der Teillösungen 1 und 2 hergestellte Beschichtungsmasse wird dann mit einem Flächengewicht von 45 g/m² auf die wie vorstehend beschriebene erst-beschichtete Trägerfolie 119 aus Polycarbonat, also auf die Nachweisschicht 118, aufgetragen und getrocknet.

### Analysegerät und Qualitätsdetektor:

In den Figuren 1 und 7 sind zwei verschiedene Ausführungsbeispiele von Analysegeräten 130 schematisch dargestellt. Dabei zeigt Figur 1 ein Ausführungsbeispiel, bei welchem Testelemente 110 in Form von Teststreifen verwendet werden, und Figur 7 zeigt ein Ausführungsbeispiel, bei welchem Testelemente 110 in Form eines Testbandes verwendet werden können. Es sei darauf hingewiesen, dass zahlreiche andere Ausführungsformen möglich sind und dass die Figuren lediglich stark schematisiert die Wirkungsweise der Analysegeräte 130 zeigen. So umfasst das Analysegerät 130 in dem in Figur 1 dargestellten Ausführungsbeispiel einen Eingabeschlitz 132, durch welchen das Testelement 110 in das Analysegerät 130 eingeschoben werden kann. Auch eine alternative Ausgestaltung, bei welche mehrere Testelemente 110 in Form von Teststreifen in dem Analysegerät 130 magaziniert aufgenommen sind, beispielsweise in einem Stangenmagazin, einem Stapelmagazin oder einem Trommelmagazin, ist denkbar. Das Testelement 110 kann beispielsweise analog zur obigen Beschreibung der Figur 8 ausgestaltet sein und kann beispielsweise ein Testfeld 116 mit einer Nachweisschicht 118 mit der Testchemie 119 und optional eine Abtrennschicht 122 umfassen. Die Aufgabe einer Probe 126, welche in Figur 1 nicht dargestellt ist, kann beispielsweise unmittelbar auf das Testfeld 116 erfolgen oder kann beispielsweise über eine Applikationsstelle 134 an einem aus dem Eingabeschlitz 132 herausragenden Ende des Testelements 110 erfolgen, gefolgt beispielsweise von einem Kapillartransport zu dem Testfeld 116. Derartige Testelemente 110 sind aus dem Stand der Technik grundsätzlich bekannt.

Weiterhin weist das Analysegerät 130 in dem dargestellten Ausführungsbeispiel mindestens einen Analytdetektor 136 und mindestens einen Qualitätsdetektor 138 auf. Diese Detektoren 136 und 138 sind in Figur 1 lediglich schematisch dargestellt. Es sei darauf hingewiesen, dass die in Figur 1 dargestellte Anordnung, beispielsweise ohne den Analytdetektor 136 und ausschließlich mit dem Qualitätsdetektor 138, auch als Ausführungsbeispiel eines separat und ohne den Analytdetektor 136 nutzbaren Qualitätsdetektors dienen kann, beispielsweise um, unabhängig von einer nachfolgenden Analytmessung, die Qualität von Teststreifen zu überprüfen.

Beispielsweise kann auf diese Weise die Qualität von Einzelteststreifen für einen visuellen Analytnachweis überprüft werden, beispielsweise von Analytteststreifen für einen Analytnachweis anhand einer vorgegebenen Farbskala. Auch andere Ausgestaltungen sind möglich.

Der Analytdetektor 136 umfasst in dem dargestellten Ausführungsbeispiel eine Analytlichtquelle 140 zur Bestrahlung der Testchemie 119 mit Analyselicht 142, in diesem Fall beispielsweise durch das Trägerelement 112 hindurch. Das Analyselicht 142 kann beispielsweise durch mindestens ein optionales Filterelement 144 optisch gefiltert werden. Weiterhin umfasst der Analytdetektor 136 in dem dargestellten Ausführungsbeispiel einen Analytphotodetektor 146 zur Aufnahme beispielsweise von Detektionslicht 148, beispielsweise gestreutem Analyselicht. Beispielsweise können auf diese Weise ein Remissionswert und/oder eine Farbänderung der Testchemie 119 beobachtet werden. Das Detektionslicht 148 kann optional durch mindestens ein Filterelement 150 gefiltert werden. Es sei darauf hingewiesen, dass auch zahlreiche andere Möglichkeiten des Analytnachweises und/oder der Ausgestaltung des Analytdetektors 136 möglich sind, beispielsweise, alternativ oder zusätzlich zu einer Messung eines Remissionswertes, die Erfassung einer Fluoreszenz. Dementsprechend wäre der Analytdetektor 136 zu modifizieren. Die Analytlichtquelle 140 und/oder der Analytphotodetektor 146 können beispielsweise als Halbleiterbauelemente ausgestaltet sein, beispielsweise als Leuchtdiode bzw. Fotodiode. Auch andere Ausgestaltungen sind möglich.

Der Qualitätsdetektor 136 umfasst eine oder mehrere Einheiten, wobei in dem dargestellten Ausführungsbeispiel gemäß Figur 1 zwei Einheiten vorgesehen sind, welche eine erste Lumineszenz in einem ersten Wellenlängenintervall und eine zweite Lumineszenz in einem zweiten Wellenlängenintervall aufweisen. So umfasst der Qualitätsdetektor 138 in dem dargestellten Ausführungsbeispiel zwei Anregungslichtquellen 152, 154, welche optional mit Filterelementen 156, 158 versehen sein können. Diese erzeugen Anregungslicht 160, 162. Es sei darauf hingewiesen, dass Anregungslicht 160, 162 unterschiedlicher Wellenlängen auch grundsätzlich durch ein und dieselbe Anregungslichtquelle 152, 154 generiert werden könnte, so dass diese Anregungslichtquellen 152, 154 auch beispielsweise zusammengefässt sein könnten, wobei beispielsweise unterschiedliche Filterelemente 156, 158 zur Erzeugung von Anregungslicht 160, 162 unterschiedlicher Wellenlängen verwendet werden könnten.

Mittels des Anregungslichts 160, 162 wird die Testchemie 119 bestrahlt, beispielsweise wiederum durch das transparente Trägerelement 112 hindurch. Diese Bestrahlung kann zeitgleich oder auch zeitversetzt erfolgen. Bei dieser Qualitätsmessung entsteht Lumineszenzlicht 164, 166, welches, optional nach Filterung durch optionale Filterelemente 168, 170, durch Qualitätsphotodetektoren 172, 174 erfasst wird. Die Elemente 152, 156, 168 und 172 bilden somit eine erste Einheit des Qualitätsdetektors 138, zur Erfassung eines ersten Lumineszenzlichts, und die Elemente 154, 158, 170, 174, eine optionale zweite Einheit zur Erfassung eines zweiten Lumineszenzlichts.

Weiterhin ist darauf hinzuweisen, dass die in Figur 1 dargestellten Strahlengänge lediglich schematisch gezeigt sind und auch auf andere Weise ausgestaltet sein können. Beispielsweise kann für jeden auf das Testelement 110 auftreffenden Strahl ein Einfallswinkel α zu einer optischen Achse 175 senkrecht zu dem Testelement 110 und/oder dem Trägerelement 112 definiert werden, und für jeden von dem Testelement 110 oder Teilen desselben ausgehenden Strahl, beispielsweise gestreute und/oder emittierte und/oder reflektierte Strahlen, ein Austrittswinkel β. In Figur 1 ist dies exemplarisch am Beispiel des Analyselichts 142 und des Detektionslichts 148 dargestellt. Typischerweise werden die Strahlengänge derart gewählt, dass für jeden einfallenden Strahl und jeden zugehörigen von dem Testelement 110 ausgehenden Strahl, beispielsweise die Strahlen 142 und 148, der Einfallswinkel α und der Austrittswinkel β verschieden gewählt werden. Beispielsweise kann das Verhältnis α > β gelten, wie in Figur 1 dargestellt, oder umgekehrt. Auf diese Weise kann beispielsweise eine diffuse Reflexion und/oder Remission erfasst werden. Auch bei Fluoreszenzmessungen wird üblicherweise Anregungslicht unter einem Winkel eingestrahlt, der von demjenigen Winkel verschieden ist, unter welchem eine zugehörige Fluoreszenz und/oder allgemein Emission erfasst wird.

Weiterhin weist das Analysegerät 130 gemäß dem Ausführungsbeispiel in Figur 1 eine Ansteuerung 176 auf, welche auch als Auswertevorrichtung 178 fungieren kann und welche beispielsweise mit dem Analydetektor 136 und/oder dem Qualitätsdetektor 138 verbunden sein kann, um diese Detektoren 136, 138 anzusteuern und/oder Signale dieser Detektoren 136, 138 auszuwerten. Die Ansteuerung 176 kann beispielsweise mindestens eine Datenverarbeitungsvorrichtung umfassen. Weiterhin kann die Ansteuerung 176 beispielsweise einen oder mehrere Datenspeicher 180 aufweisen, beispielsweise eine oder mehrere Datenbanken. Weiterhin kann die Ansteuerung 176 beispielsweise eingerichtet sein, um über eine oder mehrere Schnittstellen 182 Informationen, Daten und/oder Befehle mit einem oder mehreren weiteren Geräten auszutauschen. Weiterhin kann die Ansteuerung 176 mit mindestens einer Benutzerschnittstelle zusammenwirken, beispielsweise mit mindestens einem Anzeigenelement 184 zur Darstellung von Informationen und/oder mit einem oder mehreren Bedienelementen 186 zur Eingabe von Befehlen und/oder Informationen durch einen Benutzer.

Das Ausführungsbeispiel des Analysegeräts 130 gemäß Figur 7 kann grundsätzlich zunächst analog zu dem Ausführungsbeispiel gemäß Figur 1 ausgestaltet sein. Anstelle eines einzelnen Teststreifens als Testelement 110 wird jedoch in dem dort dargestellten Ausführungsbeispiel ein Testband verwendet, welches beispielsweise Bestandteil einer Bandkassette 188 sein kann, die beispielsweise einen Gutwickel 190 und eine Schlechtwickel 192 umfassen kann und die beispielsweise austauschbar in dem Analysegerät 130 aufgenommen sein kann. Das Testelement 110 kann in dem dargestellten Ausführungsbeispiel ein Trägerelement 112 in Form eines Trägerbands umfassen, welches von dem Gutwickel 190 zu dem Schlechtwickel 192 schrittweise gespult werden kann und welches mehrere Testfelder 116, beispielsweise analog zu dem in Figur 8 dargestellten Aufbau, mit der Testchemie 119 und optional der Abtrennschicht 122 umfassen kann.

Wiederum umfasst das Analysegerät 130 in dem dargestellten Ausführungsbeispiel einen Analytdetektor 136 und einen Qualitätsdetektor 138. Diese Detektoren 136, 138 sind in Figur 7 lediglich schematisch angedeutet. Für einen möglichen Aufbau dieser Detektoren 136, 138 kann auf die Beschreibung der Figur 1 verwiesen werden. Allerdings zeigt Figur 7, dass grundsätzlich die Detektoren 136, 138 auch räumlich versetzt zueinander angeordnet sein können. So ist in dem Ausführungsbeispiel in Figur 7 optional eine Qualitätsmessungsposition 194 und eine Analytmessungsposition 196 vorgesehen, welche räumlich versetzt in einer Bandlaufrichtung 198 angeordnet sind, derart, dass die Qualitätsmessungsposition 194 der Analytmessungsposition 196 vorgelagert ist. Auf diese Weise kann die Qualitätsmessung derart durchgeführt werden, dass eine Messung der Eigenlumineszenz der Testchemie 119 möglich ist, vor Aufgabe der Probe 126.

### Verfahren:

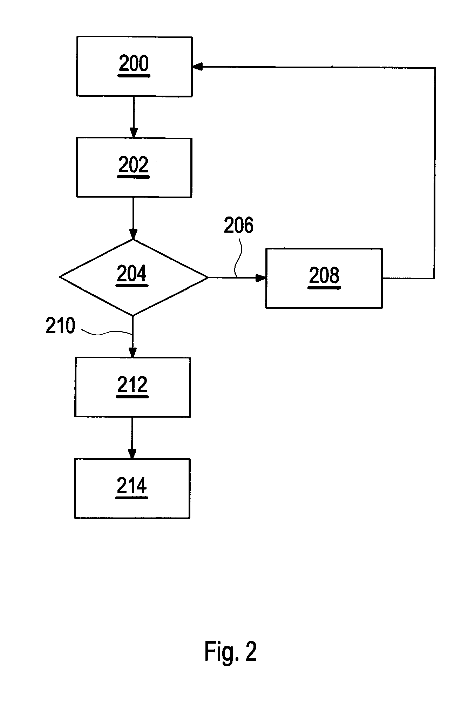

In Figur 2 ist eine mögliche Ausgestaltung eines erfindungsgemäßen Verfahrens dargestellt, welches beispielsweise mittels der Analysegeräte 130 gemäß den Ausführungsbeispielen in den Figuren 1 und 7 durchgeführt werden kann. Beispielsweise kann die Ansteuerung 176 zu diesem Zweck programmtechnisch eingerichtet sein, um das Verfahren zu realisieren.

So umfasst dieses Verfahren beispielsweise einen ersten Schritt, welche in Figur 2 auch als Start (Bezugsziffer 200) bezeichnet wird und welcher beispielsweise durch eine Eingabe eines Teststreifens und/oder durch ein Öffnen des Analysegeräts 130 und/oder durch eine Betätigung einer Starttaste erfolgen kann. So kann der Start 200 auch beispielsweise die Bereitstellung eines neuen Testelements 110 beinhalten.

Anschließend erfolgt in Schritt 202 die Durchführung einer Qualitätsmessung, bei welcher eine Eigenlumineszenz der Testchemie 119 erfasst wird, also eine Lumineszenz vor Aufgabe der Probe 126.

Anschließend kann, optional, in Verfahrensschritt 204 eine Abfrage der in Schritt 202 bestimmten Qualität des Testelements 110 erfolgen. Bei dieser Abfrage 204 kann die Qualität mit einer oder mehreren Bedingungen verglichen werden, beispielsweise indem die Qualität mit einem oder mehreren Schwellwerten verglichen wird. Optional kann dabei, wenn beispielsweise eine mangelnde Qualität festgestellt wird (Zweig 206 in Figur 2), was beispielsweise auf ein degradiertes Testelement 110 hindeuten kann, eine Warnung 208 generiert werden und/oder es kann ein Abbruch erfolgen. Dabei kann ein Benutzer beispielsweise zur Eingabe eines neuen Testelements 110 aufgefordert werden und/oder es kann ein neuer Start 200 des in Figur 2 dargestellten Verfahrens erfolgen.

Wird hingegen in Schritt 204 festgestellt, dass die Qualität für die Fortsetzung des Verfahrens ausreichend ist (Zweig 2010 in Figur 2), so kann, vorzugsweise anschließend, eine Analytmessung 212 durchgeführt werden. Bei dieser Analytemssung 212 kann ein Benutzer, beispielsweise über das Anzeigenelement 184, aufgefordert werden, die Probe 126 auf das Testelement 110 aufzugeben. Anschließend kann, beispielsweise nach einer ausreichenden Reaktionszeit für die Nachweisreaktion, mit dem Analytdetektor 136 eine Analytmessung durchgeführt werden, beispielsweise eine Messung einer Remission, wie dies grundsätzlich aus dem Stand der Technik bekannt ist. Auch andere Arten der Analytemessung sind jedoch grundsätzlich möglich.

In Verfahrensschritt 214 erfolgt schließlich eine Auswertung, welche beispielsweise eine Bestimmung, insbesondere eine Berechnung, einer Konzentration des Analyten in der Probe 126 beinhalten kann. Diese Auswertung 214 kann optional unter Verwendung der in der Qualitätsmessung 202 bestimmten Qualität durchgeführt werden. So kann beispielsweise die Auswertung 214 dahingehend erfolgen, dass die Ergebnisse der Analytmessung 212 unter Berücksichtigung der Qualität der Testchemie 119, beispielsweise einer Aktivität mindestens eines in der Testchemie 119 enthaltenen optionalen Enzyms, korrigiert werden. Beispiele einer derartigen Korrektur werden nachfolgend noch näher erläutert. Diese Korrektur kann beispielsweise durch Korrekturfaktoren und/oder eine oder mehrere Korrekturfunktionen und/oder eine oder mehrere in dem Datenspeicher 180 hinterlegte Korrekturwerte erfolgen.

### Messbeispiele:

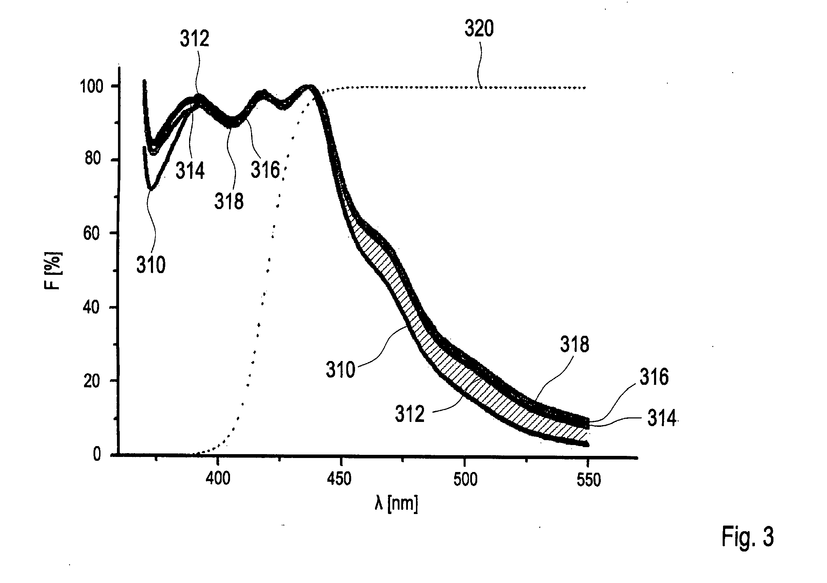

In den Figuren 3 bis 6C sind verschiedene Messbeispiele dargestellt, welche insbesondere unter Verwendung enzymatischer Testchemien 119 erzeugt wurden. So wurde, wie oben ausgeführt, im Laufe allgemeiner Untersuchungen an enzymatischen Nachweisen eine zunächst überraschende Eigenschaft derartiger Testchemien 119 mit mindestens einem Enzym festgestellt. Diese überraschende Eigenschaft besteht darin, dass mit einer Degradation eine Veränderung einer intrinsischen Lumineszenz, insbesondere einer intrinsischen Fluoreszenz, der Testchemie 119 vor einer Benetzung mit der Probe 126 einhergeht. Derartige Beobachtungen wurden zunächst an Teststreifen vor einer Benetzung verzeichnet. So wurden bei mikroskopischen Aufnahmen einer Fluoreszenz von CNAD-Teststreifen gemäß der obigen Beschreibung nach einer Einlagerung dieser Teststreifen bei unterschiedlichen Temperaturen (4 °C und 20 °C) zunächst qualitativ stark unterschiedliche Autofluoreszenzen beobachtet. So wiesen Teststreifen nach einer Lagerung bei 20 °C deutlich erhöhte Autofluoreszenz im Vergleich zu bei 4 °C gelagerten Teststreifen auf.

Aufgrund dieser Beobachtungen wurden daraufhin spezielle Untersuchungen zum Aktivitätsverlust der Testchemie 119 durchgeführt. Dabei wurden Testelemente 110 einer speziellen Belastung ("Stress") unterworfen, indem diese Testelemente 110, in diesem Fall Teststreifen, über mehrere Tage bei einer erhöhten Temperatur (60 °C) und unter erhöhter Luftfeuchtigkeit (75 % rH) gelagert wurden.

In Figur 3 sind Ergebnisse von Eigenfluoreszenzmessungen derartig behandelter Testelemente 110 dargestellt. Aufgetragen ist eine relative Fluoreszenz (normiert auf einen maximalen Wert von 100%) als Funktion der Wellenlänge. Dabei bezeichnen:
- Kurve 310: eine Eigenfluoreszenz eines Testelements 110 vor Lagerung, unmittelbar nach der Herstellung,
- Kurve 312: eine Eigenfluoreszenz am ersten Tag nach Beginn der Lagerung,
- Kurve 314: eine Eigenfluoreszenz am zweiten Tag nach Beginn der Lagerung,
- Kurve 316: eine Eigenfluoreszenz am dritten Tag nach Beginn der Lagerung, und
- Kurve 318: eine Eigenfluoreszenz am vierten Tag nach Beginn der Lagerung.

Die Spektren 310 bis 320 sind jeweils auf einen Peak bei 440 nm normiert. Die Anregung der Eigenlumineszenz in den Messungen gemäß Figur 3 erfolgte mit einer Anregungswellenlänge von 360 nm. Deutlich ist die Erhöhung der Autofluoreszenz der Teststreifen bei Wellenlängen größer als 440 nm zu erkennen. Symbolisch ist in Figur 3 daher eine Transmissionskurve einer Filtercharakteristik 320 aufgetragen, welche beispielsweise für eines der Filterelemente 168, 170 in der Anordnung des Qualitätsdetektors 138 gemäß Figur 1 verwendet werden könnte, um die erhöhte Autofluoreszenz bei Wellenlängen oberhalb von 440 nm aufzunehmen. Beispielsweise könnte hierfür ein Kantenfilter verwendet werden, welcher kommerziell erhältlich ist.

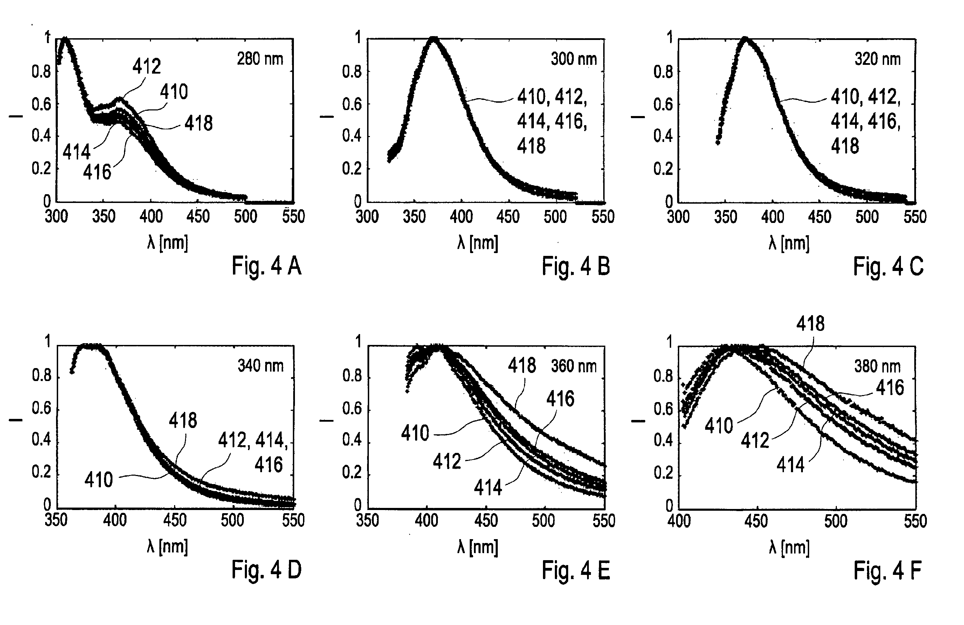

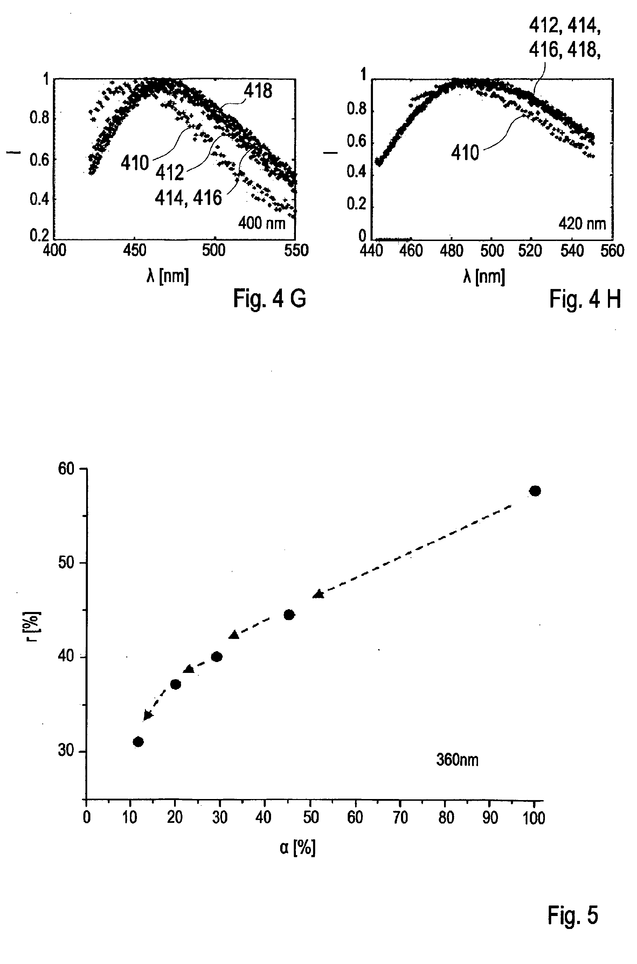

Die Messungen in Figur 3 wurden, abweichend von dem oben beschriebenen Aufbau der Testelemente 110, mit einem Trägerelement 112 in Form einer Polyethylen-Terephthalat (PET)-Folie (Melinex®) durchgeführt. Dementsprechend ist möglicherweise das Fluoreszenzsignal noch durch eine Streulichtfluoreszenz der Melinex-Trägerfolie beeinflusst. Dennoch zeigen diese ersten Messungen in Figur 3 bereits eindrucksvoll, dass eine Substanz in den Testelementen 110 unter Stressbedingungen zu einer Fluoreszenzerhöhung führt. Dennoch ließen sich die in Figur 3 gezeigten Ergebnisse auch mit leicht geänderter Rezeptur der Testchemie 119 reproduzieren und der Effekt der Melinex®-Folie ließ sich durch Verbesserung der Fluoreszenzspektroskopie reduzieren. Gleichzeitig ließ sich die Erhöhung der Autofluoreszenz der Testelemente 110 reproduzieren, was in den nachfolgenden Figuren 4A bis 4H dargestellt ist.

In den Figuren 4A bis 4H sind wiederum Fluoreszenzspektren von Testelementen 110 für verschiedene Anregungswellenlängen von 280 nm (Figur 4A) bis 420 nm (Figur 4H) dargestellt. Aufgetragen ist jeweils wieder die Fluoreszenzintensität I, normiert auf den jeweiligen Peak innerhalb des aufgenommenen Spektrums, als Funktion der Wellenlänge λ, angegeben in Nanometern. Die Anregungswellenlänge ist in den Figuren jeweils angegeben. Die Messbeispiele zeigen, dass sich bei einer Anregungswellenlänge von 360 bis 400 nm bei einer Fluoreszenz von >420 nm die deutlichsten Unterschiede nach unterschiedlicher Lagerdauer ergeben. Insbesondere steigt in diesem Bereich, wie bereits aus Figur 3 hervorgeht, die Eigenfluoreszenz mit der Lagerdauer deutlich an. Diese Eigenfluoreszenz kann somit als Kriterium für eine Degradationserkennung herangezogen werden. Insbesondere kann zur einfachen Quantifizierung einer Fluoreszenzänderung beispielsweise ein Verhältnis einer integralen Intensität bei Wellenlängen <420 nm und bei Wellenlängen >420 nm gebildet werden. So kann beispielsweise allgemein eine erste Eigenlumineszenz in einem Wellenlängenbereich <420 nm, beispielsweise in einem Wellenlängenbereich von 380 nm bis 420 nm, erfasst werde, und eine zweite Eigenlumineszenz integral in einem zweiten Wellenlängenbereich >420 nm, beispielsweise von 420 nm < Wellenlänge < 650 nm.

Dass diese Intensität der Eigenlumineszenz, beispielsweise das genannte Intensitätsverhältnis, tatsächlich einen brauchbaren Maßstab für eine Qualität der Testchemie darstellt, ist in Figur 5 gezeigt. So ist in Figur 5 das genannte Intensitätsverhältnis r in Prozent angegeben, wobei dieses Verhältnis r das Verhältnis der integralen Intensität bei Wellenlängen <420 nm zur integralen Intensität bei Wellenlängen >420 nm aufzeigt, angegeben in Prozent. Dargestellt sind hierbei Fluoreszenzmessungen bei einer Anregungswellenlänge von 360 nm.

Auf der horizontalen Achse ist als Vergleich hierzu die Aktivität der Testchemie angegeben, welche gemäß der obigen Beschreibung in einem Eluat des Testelements gemäß der oben aufgeführten Messmethode bestimmt wurden. Dabei wurde ein Eluat der Testchemie 119 erzeugt, und die Aktivität der hierin enthaltenen Enzyme über Zugabe eines Coenzyms und eines Analyten in einem laboranalytischen Verfahren bestimmt. Deutlich ist ein Zusammenhang zwischen der laboranalytisch bestimmten Aktivität und der Eigenfluoreszenz festzustellen, wobei eine Abnahme der Aktivität mit einer Zunahme der Eigenfluoreszenz bei Wellenlängen >420 nm korreliert.

In weiteren Experimenten wurde untersucht, in wie weit die erhöhte Eigenlumineszenz und insbesondere Eigenfluorenzenz direkt oder indirekt mit dem Aktivitätsabfall korreliert. Ein indirekter Zusammenhang wäre hierbei zwar eine Option, jedoch allgemein nicht wünschenswert, da in diesem Fall in einer reellen Produktsituation der stressinduzierte Veränderungsprozess möglicherweise von zwei Komponenten in gleichbleibenden Zusammenhang, beispielsweise von Rohstoff über die Verarbeitung bis hin zur Lagerung, konstant gehalten werden müsste. Gleichzeitig könnte dieser Zusammenhang konzeptionell bei der hier gewählten Belastung von 60 °C und 75 % relativer Luftfeuchtigkeit gerade zufällig bestehen, während bei genauerer Betrachtung das Enzym möglicherweise primär auf eine Temperaturbelastung und der unbekannte Stoff primär auf eine Feuchtebelastung reagieren würde.

Insofern war es wünschenswert, eine Veränderung des Enzyms direkt detektieren zu können. Aus der oben zitierten Literatur ließ sich vermuten, dass bei einer Anregung im Bereich von 340 nm bis 380 nm keine Autofluoreszenz des Enzyms selbst zu erwarten war. Zusätzlich suggerierten ähnliche Stressversuche, wenngleich nicht am identischen Enzym, in der Literatur, dass eine denkbare Fluoreszenzänderung unter Stress, wenn überhaupt, dann zu einer Abnahme und nicht zu einer Zunahme der Autofluoreszenz nach Belastung führen sollte.

Um den fluoreszierenden Stoff zu identifizieren, wurden daher im Labor Testelemente 110 hergestellt, die lediglich das Trägerelement 112 und das oben genannte, in der Literatur enthaltene Gantrez® S-97 (ein Copolymer aus Maleinanhydrid und Methyloinylether) als Verdicker, sowie jeweils nur einen der anderen Einsatzstoffe beinhalteten. Auch das reine Trägerelement (Pokalon®) als Basismaterial wurde vermessen.

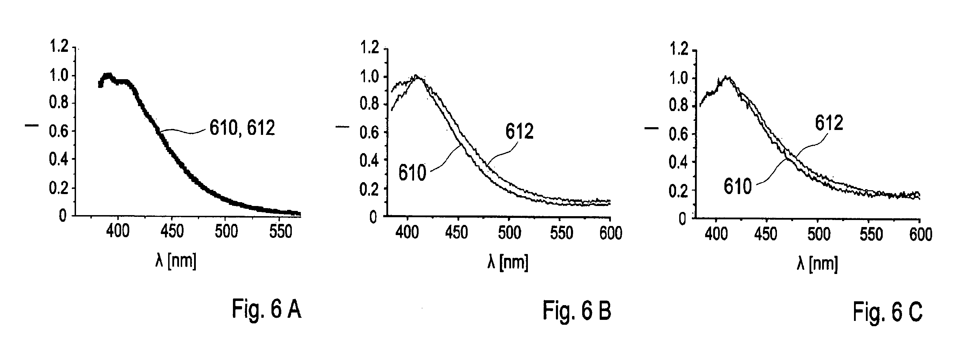

Die Ergebnisse dieser Vergleichsmessungen sind in den Figuren 6A bis 6C dargestellt. Aufgetragen ist auf der vertikalen Achse jeweils die Eigenfluoreszenz bei einer Anregungswellenlänge von 360 nm, normiert auf das im beobachteten Wellenlängenintervall angegebene Maximum, und auf der horizontalen Achse die Detektionswellenlänge λ in Nanometern. Dabei zeigen die Kurven 610 jeweils Fluoreszenzen vor einer Lagerung, und die Kurven 612 Fluoreszenzen nach fünf Tagen, also am vierten Tag nach der Einlagerung, bei einer Belastung von 60 °C und 75 % relativer Luftfeuchtigkeit.

In Figur 6A ist die Fluoreszenz der Pokalon-Folie, welche als Trägerelement 112 verwendet wird, dargestellt. Es zeigt sich deutlich, dass Pokalon keine Fluoreszenzunterschiede vor und nach der Belastung aufweist, da die Kurven 610 und 612 zusammenfallen.

In Figur 6B ist eine Messung an einer Pokalon-Folie mit Gantrez® sowie dem Enzym Glucose-Dehydrogenase gemäß der obigen Beschreibung gezeigt. Aus dieser Darstellung ergibt sich, dass insbesondere im Wellenlängenbereich >420 nm, die Eigenfluoreszenz nach der Belastung (Kurve 612) im Vergleich zu einem Ursprungszustand deutlich angestiegen ist.

In Figur 6C sind Messungen an Elementen mit einer Pokalon®-Folie, Gantrez® und dem Coenzym cNAD gezeigt. Auch hier lässt sich wiederum ein leichter Anstieg der Eigenlumineszenz in einem Wellenlängenbereich >420 nm nach Belastung feststellen.

Die Experimente zeigen somit, dass tatsächlich das Enzym sowie ggf. das Coenzym selbst die Erhöhung der Eigenlumineszenz der Testchemie 119 verursacht. Diese Experimente gaben Anlass zur Idee, diese erhöhte Eigenlumineszenz nach Belastung zu nutzen, um eine Degradation der Testchemie 119, insbesondere eine Enzymdegradation, im Testelement 110 unmittelbar mit den Eigenschaften der Testchemie 119, insbesondere des Enzyms, nachweisen zu können. Diese Erhöhung der Eigenlumineszenz, insbesondere der Eigenfluoreszenz, wird bei Testelementen 110, beispielsweise Teststreifen, vor einer Benetzung mit der Probe 126 festgestellt, d.h. bereits vor Benutzung durch einen Benutzer. Ein System zum Nachweis dieser Eigenlumineszenz bedarf, wie anhand der Figuren 1 und 7 exemplarisch dargestellt, in der Regel lediglich eines Photodetektors welcher, z.B. durch geeignete Wahl der Filter, nicht auf das Anregungslicht, jedoch um so mehr auf das bei der Anregung entstehende Lumineszenzlicht und insbesondere Fluoreszenzlicht reagiert. Beispielsweise könnte eine Fotodiode mit einem geeigneten Filter die erhöhte Eigenlumineszenz, insbesondere Eigenfluoreszenz, detektieren. Wie oben bereits erwähnt, können grundsätzlich auch mehrere Qualitätsphotodetektoren 172, 174 verwendet werden, von denen eine beispielsweise das Fluoreszenzlicht von 380 nm bis 420 nm detektiert und die andere Fotodiode beispielsweise das Fluoreszenzlicht zwischen 420 nm und 650 nm. Aus den Meßsignalen dieser Fotodioden oder allgemein Photodetektoren könnt dann beispielsweise eine Differenz gebildet werden oder, alternativ oder zusätzlich, könnten diese Signale ins Verhältnis gesetzt werden, um auf diese Weise beispielsweise eine Maßzahl für die Fluoreszenzaktivität und/oder eine Qualität der Testchemie 119 zu erhalten. Um aus der Maßzahl auf eine Aktivität der Testchemie 119, beispielsweise eine Enzymaktivität, schließen zu können, könnte beispielsweise eine Korrekturkurve, beispielsweise eine Eichkurve analog zu der in Figur 5 dargestellten Kurve, verwendet werden. Es ist auch beispielsweise denkbar, eine Differenz der beiden Signale zu bilden und diese Differenz dann beispielsweise zum Mittelwert der beiden Signale ins Verhältnis zu setzten. Viele andere Konstellationen sind denkbar, beispielsweise eine Referenzierung zum Anregungslicht, beispielsweise einer Leerwertremission oder ähnliche Referenzierungen.

**Bezugszeichenliste**

| | | | |
|---|---|---|---|
| 110 | Testelement | 175 | optische Achse |
| 112 | Trägerelement | 176 | Ansteuerung |
| 113 | eingestrahltes Licht | 178 | Auswertevorrichtung |
| 114 | detektierbares Licht | 180 | Datenspeicher |
| 116 | Testfeld | 182 | Schnittstelle |
| 118 | Nachweisschicht | 184 | Anzeigeelement |
| 119 | Testchemie | 186 | Bedienelement |
| 120 | Nachweisseite | 188 | Bandkassette |
| 122 | Abtrennschicht | 190 | Gutwickel |
| 124 | Testfeldoberfläche | 192 | Schlechtwickel |
| 126 | Probe | 194 | Qualitätsmessungsposition |
| 128 | Aufgabeseite | 196 | Analytmessungsposition |
| 130 | Analysegerät | 198 | Bandlaufrichtung |
| 132 | Eingabeschlitz | 200 | Start |
| 134 | Applikationsstelle | 202 | Qualitätsmessung |
| 136 | Analytdetektor | 204 | Abfrage Qualität |
| 138 | Qualitätsdetektor | 206 | mangelnde Qualität |
| 140 | Analytlichtquelle | 208 | Warnung, Abbruch |
| 142 | Analyselicht | 210 | ausreichende Qualität |
| 144 | Filterelement | 212 | Analytmessung |
| 146 | Analytphotodetektor | 214 | Auswertung |
| 148 | Detektionslicht | 310 | vor Lagerung |
| 150 | Filterelement | 312 | 1. Tag |
| 152 | Anregungslichtquelle | 314 | 2. Tag |
| 154 | Anregungslichtquelle | 316 | 3. Tag |
| 156 | Filterelement | 318 | 4. Tag |
| 158 | Filterelement | 320 | Filtercharakteristik |
| 160 | Anregungslicht | 410 | vor Lagerung |
| 162 | Anregungslicht | 412 | 1. Tag |
| 164 | Lumineszenzlicht | 414 | 2. Tag |
| 166 | Lumineszenzlicht | 416 | 3. Tag |
| 168 | Filterelemente | 418 | 4. Tag |
| 170 | Filterelemente | 610 | vor Lagerung |
| 172 | Qualitätsphotodetektor | 612 | 4. Tag |
| 174 | Qualitätsphotodetektor | | |

## Patentansprüche

1. Analysegerät (130) zum Nachweis mindestens eines Analyten in einer Probe (126), insbesondere zum Nachweis von Blutglukose,
- wobei das Analysegerät (130) mindestens ein Testelement (110) aufweist, wobei das Testelement (110) mindestens eine Testchemie (119) aufweist, wobei das Testelement (110) mindestens ein Testfeld (116) aufweist, wobei das Testfeld (116) ein Bereich ist, in dem mindestens eine zusammenhängende Schicht der Testchemie (119) auf ein Trägerelement (112) aufgebracht oder in ein Trägerelement (112) eingebracht ist, wobei das Analysegerät (130) eingerichtet ist, um mittels des Testelements (110) mindestens eine Analytmessung durchzuführen, wobei die Testchemie (119) eine Substanz oder ein Substanzgemisch ist, welches eingerichtet ist, um bei Anwesenheit des Analyten mindestens eine nachweisbare veränderliche Eigenschaft zu verändern, insbesondere eine elektrische und/oder optische Eigenschaft, wobei bei der Analytmessung die mindestens eine durch Anwesenheit des Analyten veränderliche Eigenschaft der mindestens einen Testchemie (119) des Testelements (110) erfasst wird, und
- wobei das Analysegerät (130) weiterhin eingerichtet ist, um mindestens eine Qualitätsmessung an der Testchemie (119) durchzuführen, wobei bei der Qualitätsmessung mindestens eine Eigenlumineszenz der Testchemie (119) erfasst wird und aus der Eigenlumineszenz auf eine Qualität der Testchemie (119) geschlossen wird, wobei die Qualität der Testchemie (119) eine Information ist, welche Aufschluss über einen Zustand der Testchemie (119) gibt, wobei die Qualität mindestens eine Qualitätsinformation umfasst, welche eine Degradation oder einen Alterungszustand der Testchemie (119) quantifiziert,
wobei das Analysegerät (130) eingerichtet ist, um die Qualitätsmessung mindestens einmal vor der Analytmessung durchzuführen, wobei das Analysegerät (130) eingerichtet ist, um entsprechend der erfassten Qualität mindestens eine Aktion durchzuführen, ausgewählt aus der Gruppe bestehend aus: einer Ausgabe eines Hinweises an einen Benutzer, insbesondere eines Warnhinweises; einer Weitergabe mindestens einer Information über die Qualität an mindestens ein weiteres Gerät; einer Abspeicherung mindestens einer Information über die Qualität in einem Datenspeicher (180); einer Verhinderung oder Ermöglichung der Analytmessung; einer Berücksichtigung oder Nichtberücksichtigung einer bereits erfolgten Analytmessung; einer Berechnung einer Konzentration des Analyten in der Probe aus der erfassten Eigenschaft unter Berücksichtigung der Qualität der Testchemie, insbesondere unter Verwendung einer die Qualität berücksichtigenden Korrektur.

2. Analysegerät (130) nach dem vorhergehenden Anspruch, wobei das Analysegerät (130) eingerichtet ist, um auf eine Degradation des Testelements (110) zu schließen, wenn die Eigenlumineszenz mindestens eine vorgegebene Schwelle überschreitet.

3. Analysegerät (130) nach einem der vorhergehenden Ansprüche, wobei das Analysegerät (130) eingerichtet ist, um eine Berechnung einer Konzentration des Analyten in der Probe (126) aus der erfassten Eigenschaft unter Berücksichtigung der Qualität der Testchemie (119) durchzuführen, insbesondere unter Verwendung einer die Qualität berücksichtigenden Korrektur.

4. Analysegerät (130) nach einem der vorhergehenden Ansprüche, wobei das Analysegerät (130) weiterhin mindestens einen optischen Qualitätsdetektor (138) umfasst und eingerichtet ist, um bei der Qualitätsmessung mittels des optischen Qualitätsdetektors (138) die Messung der Eigenlumineszenz der Testchemie (119) durchzuführen, wobei das Analysegerät (130) eingerichtet ist, um mittels des optischen Qualitätsdetektors (138) die Eigenlumineszenz in mindestens zwei verschiedenen Wellenlängenbereichen zu erfassen, insbesondere mindestens eine erste Eigenlumineszenz oder mindestens ein erstes Eigenlumineszenzspektrum in einem ersten Wellenlängenintervall und mindestens eine zweite Eigenlumineszenz oder mindestens ein zweites Eigenlumineszenzspektrum in mindestens einem zweiten Wellenlängenintervall.

5. Analysegerät (130) nach dem vorhergehenden Anspruch, wobei das Analysegerät (130) eingerichtet ist, um aus der Eigenlumineszenz in den mindestens zwei verschiedenen Wellenlängenbereichen mindestens eine die Qualität charakterisierende Qualitätskennzahl zu berechnen, insbesondere durch Quotientenbildung und/oder Bildung einer Linearkombination.

6. Analysegerät (130) nach einem der vorhergehenden Ansprüche, weiterhin umfassend eine Auswertevorrichtung (178), wobei die Auswertevorrichtung (178) eingerichtet ist, um die Qualität mit mindestens einer Bedingung zu vergleichen, insbesondere um die Qualität mit mindestens einem Schwellwert zu vergleichen.

7. Analysegerät (130) nach einem der vorhergehenden Ansprüche, wobei das Analysegerät (130) eingerichtet ist, um entsprechend der erfassten Qualität mindestens eine Aktion durchzuführen, insbesondere eine Aktion ausgewählt aus der Gruppe bestehend aus: einer Ausgabe eines Hinweises an einen Benutzer, insbesondere eines Warnhinweises; einer Weitergabe mindestens einer Information über die Qualität an mindestens ein weiteres Gerät; einer Abspeicherung mindestens einer Information über die Qualität in einem Datenspeicher (180); einer Verhinderung oder Ermöglichung der Analytmessung; einer Berücksichtigung oder Nichtberücksichtigung einer bereits erfolgten Analytmessung.

8. Analysegerät (130) nach einem der vorhergehenden Ansprüche, wobei die Testchemie (119) mindestens ein Enzym umfasst, insbesondere ein Enzym ausgewählt aus der Gruppe bestehend aus: einer Oxidase; einer Dehydrogenase, insbesondere einer Dehydrogenase ausgewählt aus der Gruppe bestehend aus Glucose 6-Phosphat-Dehydrogenase (EC 1.1.1.49), NAD-abhängiger Cholesterol-Dehydrogenase (EC 1.1.1.62), FAD-abhängige Glucose-Dehydrogenase (EC 1.1.99.10) und PQQabhängige Glucose-Dehydrogenase (EC 1.1.5.2).

9. Analysegerät (130) nach dem vorhergehenden Anspruch, wobei das Enzym ausgewählt ist aus der Gruppe bestehend aus: Glucose-Dehydrogenase (E.C.1.1.1.47); Lactat-Dehydrogenase (E.C.1.1.1.27, 1.1.1.28); Malat-Dehydrogenase (E.C.1.1.1.37); Glycerin-Dehydrogenase (E.C.1.1.1.6); Alkohol-Dehydrogenase (E.C.1.1.1.1); alpha-Hydroxybutyrat-Dehydrogenase; Sorbitol-Dehydrogenase; Aminosäure-Dehydrogenase, insbesondere L-Aminosäure-Dehydrogenase (E.C.1.4.1.5); Glucoseoxidase (E.C.1.1.3.4); Cholesterinoxidase (E.C.1.1.3.6); Aminotransferasen, insbesondere Aspartat oder Alanin Aminotransferase; 5'-Nukleotidase; Creatin-Kinase; Glucose 6-Phosphat-Dehydrogenase (EC 1.1.1.49); NAD-abhängiger Cholesterol-Dehydrogenase (EC 1.1.1.62); FAD-abhängiger Glucose-Dehydrogenase (EC 1.1.99.10); PQQ-abhängiger Glucose-Dehydrogenase (EC 1.1.5.2).

10. Analysegerät (130) nach einem der beiden vorhergehenden Ansprüche, wobei die Qualität mindestens eine Information über eine Aktivität des Enzyms umfasst.

11. Verfahren zum Nachweis mindestens eines Analyten in einer Probe (126), insbesondere unter Verwendung eines Analysegeräts (130) nach einem der vorhergehenden Ansprüche wobei das Verfahren folgende Schritte umfasst:
- mindestens eine Analytmessung, wobei bei der Analytmessung mindestens eine durch Anwesenheit des Analyten veränderliche Eigenschaft mindestens einer Testchemie (119) eines Testelements (110) erfasst wird, insbesondere eine elektrische und/oder optische Eigenschaft, wobei das Testelement (110) mindestens ein Testfeld (116) aufweist, wobei das Testfeld (116) ein Bereich ist, in dem mindestens eine zusammenhängende Schicht der Testchemie (119) auf ein Trägerelement (112) aufgebracht oder in ein Trägerelement (112) eingebracht ist, und
- mindestens eine Qualitätsmessung an der Testchemie (119), wobei bei der Qualitätsmessung mindestens eine Eigenlumineszenz der Testchemie (119) erfasst wird und aus der Eigenlumineszenz auf eine Qualität der Testchemie (119) geschlossen wird, wobei die Qualität mindestens eine Qualitätsinformation umfasst, welche eine Degradation oder einen Alterungszustand der Testchemie (119) quantifiziert,
wobei entsprechend der erfassten Qualität mindestens eine Aktion durchgeführt wird, ausgewählt aus der Gruppe bestehend aus: einer Ausgabe eines Hinweises an einen Benutzer, insbesondere eines Warnhinweises; einer Weitergabe mindestens einer Information über die Qualität an mindestens ein weiteres Gerät; einer Abspeicherung mindestens einer Information über die Qualität in einem Datenspeicher (180); einer Verhinderung oder Ermöglichung der Analytmessung; einer Berücksichtigung oder Nichtberücksichtigung einer bereits erfolgten Analytmessung; einer Berechnung einer Konzentration des Analyten in der Probe aus der erfassten Eigenschaft unter Berücksichtigung der Qualität der Testchemie, insbesondere unter Verwendung einer die Qualität berücksichtigenden Korrektur.

12. Verwendung eines Analysegeräts (130) nach einem der vorhergehenden, ein Analysegerät (130) betreffenden Ansprüche zur Vermeidung von Analytmessungen unter Verwendung von Testelementen (110) mit degradierter Testchemie (119).

## Claims

1. Analytical apparatus (130) for detecting at least one analyte in a sample (126), in particular for detecting blood glucose,
- wherein the analytical apparatus (130) contains at least one test element (110), the test element (110) containing at least one test chemical (119), the test element (110) having at least one test field (116), the test field (116) being an area in which at least one coherent layer of the test chemical (119) is applied to a carrier element (112) or incorporated into a carrier element (112), the analytical apparatus (130) being equipped, by means of the test element (110), to carry out at least one analyte measurement, wherein the test chemical (119) is a substance or a substance mixture, which is equipped, in presence of the analyte, to change at least one detectable changeable property, in particular an electrical and/or optical property, wherein in the analyte measurement the at least one property changeable by presence of the analyte of the at least one test chemical (119) of the test element (110) is recorded, and
- wherein the analytical apparatus (130) is furthermore equipped to carry out at least one quality measurement on the test chemical (119), wherein in the quality measurement at least an intrinsic luminescence of the test chemical (119) is recorded and from the intrinsic luminescence a quality of the test chemical (119) is concluded, wherein the quality of the test chemical (119) is information which provides information about a state of the test chemical (119), wherein the quality comprises at least one item of quality information which quantifies a degradation or an aging state of the test chemical (119),
the analytical apparatus (130) being equipped to carry out the quality measurement at least once before the analyte measurement, the analytical apparatus (130) being equipped, corresponding to the recorded quality, to carry out at least one action, selected from the group consisting of: an output of a message to a user, in particular a warning message; a dissemination of at least one item of information about the quality to at least a further device; a storage of at least one item of information about the quality in a data store (180); a prevention or facilitation of the analyte measurement; a consideration or non-consideration of an analyte measurement already carried out; a calculation of a concentration of the analyte in the sample from the recorded property, taking into consideration the quality of the test chemical, in particular using a correction taking into consideration the quality.

2. Analytical apparatus (130) according to the preceding claim, wherein the analytical apparatus (130) is equipped to draw conclusions as to a degradation of the test element (110) if the intrinsic luminescence exceeds at least one predetermined threshold.

3. Analytical apparatus (130) according to one of the preceding claims, wherein the analytical apparatus (130) is equipped to carry out a calculation of a concentration of the analyte in the sample (126) from the recorded property, taking into consideration the quality of the test chemical (119), in particular using a correction taking into consideration the quality.

4. Analytical apparatus (130) according to one of the preceding claims, wherein the analytical apparatus (130) furthermore comprises at least one optical quality detector (138) and is equipped in the quality measurement by means of the optical quality detector (138) to carry out the measurement of the intrinsic luminescence of the test chemical (119), wherein the analytical apparatus (130) is equipped, by means of the optical quality detector (138), to record the intrinsic luminescence in at least two different wavelength regions, in particular at least a first intrinsic luminescence or at least a first intrinsic luminescence spectrum in a first wavelength interval and at least a second intrinsic luminescence or at least a second intrinsic luminescence spectrum in at least a second wavelength interval.

5. Analytical apparatus (130) according to the preceding claim, wherein the analytical apparatus (130) is equipped, from the intrinsic luminescence in the at least two different wavelength regions, to calculate at least a quality index characterizing the quality, in particular by quotient formation and/or formation of a linear combination.

6. Analytical apparatus (130) according to one of the preceding claims, furthermore comprising an evaluation device (178), wherein the evaluation device (178) is equipped to compare the quality with at least one condition, in particular to compare the quality with at least a threshold value.

7. Analytical apparatus (130) according to one of the preceding claims, wherein the analytical apparatus (130) is equipped, corresponding to the recorded quality, to carry out at least one action, in particular an action selected from the group consisting of: an output of a message to a user, in particular a warning message; a dissemination of at least one item of information about the quality to at least a further device; storage of at least one item of information about the quality in a data store (180); a prevention or facilitation of the analyte measurement; a consideration or non-consideration of an analyte measurement already carried out.

8. Analytical apparatus (130) according to one of the preceding claims, wherein the test chemical (119) comprises at least one enzyme, in particular an enzyme selected from the group consisting of: an oxidase; a dehydrogenase, in particular a dehydrogenase selected from the group consisting of glucose 6-phosphate dehydrogenase (EC 1.1.1.49), NAD-dependent cholesterol dehydrogenase (EC 1.1.1.62), FAD-dependent glucose dehydrogenase (EC 1.1.99.10) and PQQ-dependent glucose dehydrogenase (EC 1.1.5.2).

9. Analytical apparatus (130) according to the preceding claim, wherein the enzyme selected is from the group consisting of: glucose dehydrogenase (E.C.1.1.1.47); lactate dehydrogenase (E.C.1.1.1.27, 1.1.1.28); malate dehydrogenase (E.C.1.1.1.37); glycerol dehydrogenase (E.C.1.1.1.6); alcohol dehydrogenase (E.C.1.1.1.1); alpha-hydroxybutyrate dehydrogenase; sorbitol dehydrogenase; amino acid dehydrogenase, in particular L-amino acid dehydrogenase (E.C.1.4.1.5); glucose oxidase (E.C.1.1.3.4); cholesterol oxidase (E.C.1.1.3.6); aminotransferases, in particular aspartate or alanine aminotransferase; 5'-nucleotidase; creatine kinase; glucose 6-phosphate dehydrogenase (EC 1.1.1.49); NAD-dependent cholesterol dehydrogenase (EC 1.1.1.62); FAD-dependent glucose dehydrogenase (EC 1.1.99.10); PQQ-dependent glucose dehydrogenase (EC 1.1.5.2).

10. Analytical apparatus (130) according to one of the two preceding claims, wherein the quality comprises at least one item of information about an activity of the enzyme.

11. Method for detecting at least one analyte in a sample (126), in particular using an analytical apparatus (130) according to one of the preceding claims, wherein the method comprises the following steps:
- at least one analyte measurement, wherein in the analyte measurement at least a property of at least one test chemical (119) of a test element (110) changeable by presence of the analyte is recorded, in particular an electrical and/or optical property, the test element (110) having at least one test field (116), the test field (116) being an area in which at least one coherent layer of the test chemical (119) is applied to a carrier element (112) or incorporated into a carrier element (112), and
- at least a quality measurement on the test chemical (119), wherein in the quality measurement at least an intrinsic luminescence of the test chemical (119) is recorded and from the intrinsic luminescence a quality of the test chemical (119) is concluded, wherein the quality comprises at least one item of quality information which quantifies a degradation or an aging state of the test chemical (119),
corresponding to the recorded quality at least one action being carried out, selected from the group consisting of: an output of a message to a user, in particular a warning message; a dissemination of at least one item of information about the quality to at least a further device; a storage of at least one item of information about the quality in a data store (180); prevention or facilitation of the analyte measurement; a consideration or non-consideration of an analyte measurement already carried out; a calculation of a concentration of the analyte in the sample from the recorded property, taking into consideration the quality of the test chemical, in particular using a correction taking into consideration the quality.

12. Use of an analytical apparatus (130) according to one of the above claims, relating to an analytical apparatus (130), for avoiding analyte measurements using test elements (110) containing degraded test chemical (119).

## Revendications

1. Instrument d'analyse (130) pour un dépistage d'au moins un analyte dans un échantillon (126), en particulier pour le dépistage de glucose dans le sang,
- l'instrument d'analyse (130) présentant au moins un élément de test (110), l'élément de test (110) contenant au moins un produit chimique de test (119), l'élément de test (110) présentant au moins un champ de test (116), le champ de test (116) étant une zone dans laquelle au moins une couche cohésive de produit chimique de test (119) est appliquée sur un élément porteur (112) ou intégrée dans un élément porteur (112), l'instrument d'analyse (130) étant conçu pour réaliser, au moyen de l'élément de test (110), au moins une mesure d'analyte, le produit chimique de test (119) étant une substance ou un mélange de substances qui est conçu pour, en présence de l'analyte, modifier au moins une propriété variable dépistable, en particulier une propriété électrique et/ou optique, l'au moins une propriété variable du fait de la présence de l'analyte de l'au moins un produit chimique de test (119) de l'élément de test (110) étant relevée lors de la mesure d'analyte, et
- l'instrument d'analyse (130) étant en outre conçu pour réaliser au moins une mesure de qualité du produit chimique de test (119), sachant que, lors de la mesure de qualité, au moins une luminescence propre du produit chimique de test (119) est relevée et que, à partir de la luminescence propre, on conclut à une qualité du produit chimique de test (119), la qualité du produit chimique de test (119) étant une information qui donne des renseignements sur un état du produit chimique de test (119), la qualité comprenant au moins une information qualitative qui quantifie une dégradation ou un état de vieillissement du produit chimique de test (119),
l'instrument d'analyse (130) étant conçu pour réaliser la mesure de qualité au moins une fois avant la mesure d'analyte, l'instrument d'analyse (130) étant conçu pour réaliser, en fonction de la qualité relevée, au moins une action sélectionnée dans le groupe composé des actions suivantes : édition d'une information vers un utilisateur, en particulier d'un avertissement, retransmission d'au moins une information sur la qualité à au moins un autre instrument ; sauvegarde d'au moins une information sur la qualité dans une mémoire de données (180) ; empêchement ou autorisation de la mesure d'analyte ; prise en compte ou non-prise en compte d'une mesure d'analyte déjà effectuée ; calcul d'une concentration de l'analyte dans l'échantillon à partir de la propriété relevée en tenant compte de la qualité du produit chimique de test, en particulier en utilisant une correction tenant compte de la qualité.

2. Instrument d'analyse (130) selon la revendication précédente, dans lequel l'instrument d'analyse (130) est conçu pour conclure à une dégradation de l'élément de test (110) si la luminescence propre dépasse au moins un seul prédéfini.

3. Instrument d'analyse (130) selon l'une quelconque des revendications précédentes, dans lequel l'instrument d'analyse (130) est conçu pour effectuer un calcul d'une concentration de l'analyte dans l'échantillon (126) à partir de la propriété relevée en tenant compte de la qualité du produit chimique de test (119), en particulier en utilisant une correction tenant compte de la qualité.

4. Instrument d'analyse (130) selon l'une quelconque des revendications précédentes, dans lequel l'instrument d'analyse (130) comprend en outre au moins un détecteur de qualité optique (138) et est conçu pour réaliser la mesure de luminescence propre du produit chimique de test (119) lors de la mesure de qualité au moyen du détecteur de qualité optique (138), l'instrument d'analyse (130) étant conçu pour relever, au moyen du détecteur de qualité optique (138), la luminescence propre dans au moins deux plages de longueurs d'ondes différentes, en particulier au moins une première luminescence propre ou au moins un premier spectre de luminescence propre dans un premier intervalle de longueurs d'ondes et au moins une seconde luminescence propre ou au moins un second spectre de luminescence propre dans au moins un second intervalle de longueurs d'ondes.

5. Instrument d'analyse (130) selon l'une quelconque des revendications précédentes, dans lequel l'instrument d'analyse (130) est conçu pour, à partir de la luminescence propre dans les au moins deux plages de longueurs d'ondes différentes, calculer au moins une caractéristique de qualité caractérisant la qualité, en particulier en établissant des quotients et/ou en établissant une combinaison linéaire.

6. Instrument d'analyse (130) selon l'une quelconque des revendications précédentes, comprenant en outre un dispositif d'exploitation (178), le dispositif d'exploitation (178) étant conçu pour comparer la qualité à au moins une condition, en particulier pour comparer la qualité à au moins une valeur seuil.

7. Instrument d'analyse (130) selon l'une quelconque des revendications précédentes, dans lequel l'instrument d'analyse (130) est conçu pour, en fonction de la qualité relevée, réaliser au moins une action, en particulier une action sélectionnée dans le groupe composé des actions suivantes : édition d'une information vers un utilisateur, en particulier d'un avertissement, retransmission d'au moins une information sur la qualité à au moins un autre instrument ; sauvegarde d'au moins une information sur la qualité dans une mémoire de données (180) ; empêchement ou autorisation de la mesure d'analyte ; prise en compte ou non-prise en compte d'une mesure d'analyte déjà effectuée.

8. Instrument d'analyse (130) selon l'une quelconque des revendications précédentes, dans lequel le produit chimique de test (119) comprend au moins une enzyme sélectionnée dans le groupe composé des éléments suivants : une oxydase, une déshydrogénase, en particulier une déshydrogénase enzyme sélectionnée dans le groupe composé des éléments suivants : glucose 6-phosphate-déshydrogénase (E.C.1.1.1.49), cholestérol NAD-dépendant-déshydrogénase (E.C.1.1.1.62), glucose FAD-dépendant-déshydrogénase (E.C.1.1.99.10) et glucose PQQ-dépendant-déshydrogénase (E.C.1.1.5.2).

9. Instrument d'analyse (130) selon l'une quelconque des revendications précédentes, dans lequel l'enzyme est sélectionnée dans le groupe composé des éléments suivants : glucose-déshydrogénase (E.C.1.1.1.47) ; lactate-déshydrogénase (E.C.1.1.1.27, 1.1.1.28) ; malate-déshydrogénase (E.C.1.1.1.37) glycérine-déshydrogénase (E.C.1.1.1.6) ; alcool-déshydrogénase (E.C.1.1.1.1) ; alpha-hydroxybutyrate-déshydrogénase ; sorbitol-déshydrogénase ; acide aminé-déshydrogénase, en particulier acide L-aminé-déshydrogénase (E.C.1.4.1.5) ; glucose-oxydase (E.C.1.1.3.4) ; cholestérol-oxydase (E.C.1.1.3.6) ; aminotransférases, en particulier aspartate ou alinine aminotransférase ; 5'-nucléotidases ; créatine-kinase ; glucose 6-phosphate-déshydrogénase (E.C.1.1.1.49) ; cholestérol NAD-dépendant-déshydrogénase (E.C.1.1.62) ; glucose FAD-dépendant-déshydrogénase (E.C.1.1.99.10) ; glucose PQQ-dépendant-déshydrogénase (E.C.1.1.5.2).

10. Instrument d'analyse (130) selon l'une quelconque des deux revendications précédentes, dans lequel la qualité comprend au moins une information sur une activité de l'enzyme.

11. Procédé de dépistage d'au moins un analyte dans un échantillon (126), en particulier en utilisant un instrument d'analyse (130) selon l'une quelconque des revendications précédentes, ce procédé comprenant les étapes suivantes :
- au moins une mesure d'analyte, sachant que, lors de la mesure d'analyte, au moins une propriété variable du fait de la présence de l'analyte d'au moins un produit chimique de test (119) d'un élément de test (110) est relevée, en particulier une propriété électrique et/ou optique, l'élément de test (110) présentant au moins un champ de test (116), le champ de test (116) étant une zone dans laquelle au moins une couche cohésive de produit chimique de test (119) est appliquée sur un élément porteur (112) ou intégrée dans un élément porteur (112), et
- au moins une mesure de qualité du produit chimique de test (119), sachant que, lors de la mesure de qualité, une luminescence propre du produit chimique de test (119) est relevée et que, à partir de la luminescence propre, on conclut à une qualité du produit chimique de test (119), la qualité englobant au moins une information qualitative qui quantifie une dégradation ou un état de vieillissement du produit chimique de test (119),
étant réalisée, en fonction de la qualité relevée, au moins une action sélectionnée dans le groupe composé des actions suivantes : édition d'une information vers un utilisateur, en particulier d'un avertissement, retransmission d'au moins une information sur la qualité à au moins un autre instrument ; sauvegarde d'au moins une information sur la qualité dans une mémoire de données (180) ; empêchement ou autorisation de la mesure d'analyte ; prise en compte ou non-prise en compte d'une mesure d'analyte déjà effectuée ; calcul d'une concentration de l'analyte dans l'échantillon à partir de la propriété relevée en tenant compte de la qualité du produit chimique de test, en particulier en utilisant une correction tenant compte de la qualité.

12. Utilisation d'un instrument d'analyse (130) selon l'une quelconque des revendications précédentes concernant un instrument d'analyse (130) pour éviter de faire des mesures d'analyte en utilisant des éléments de test (110) dont le produit chimique de test (119) est dégradé.
